# EUROPEAN PATENT APPLICATION

(11) **EP 1 882 687 A1**
(43) Date of publication of application: **30.01.2008**
(21) Application number: 06015724.5
(22) Date of filing: 27.07.2006
(51) Int. Cl.: C07D 213/56, C07D 213/65, C07D 213/64, C07D 213/61, A61K 31/44, A61P 25/00, A61P 29/00

(54) **Heterocyclic compounds useful as vanilloid receptor antagonists and pharmaceutical compositions containing the same**

(71) Applicant: Amorepacific Corporation, Seoul 140-777 (KR)
(72) Inventor: Shin, Song Seok, Yongin-si Gyeonggi-do 449-959 (KR); Kim, Sun-Young, Seocho-gu Seoul 137-873 (KR); Kim, Jin Kwan, Suwon-si Gyeonggi-do 441-350 (KR); Lee, Li-Wah, Gangnam-gu Seoul 135-230 (KR); Woo, Byoung Young, Cheoin-gu Yongin-si Gyeonggi-do 449-722 (KR); Lim, Kyung Min, Youngtong-ku Suwon-si Kyunggi-do 443-725 (KR); Jeong, Yeon Su, Yongin-si Kyunggi-do 449-749 (KR); Choi, Jin Kyu, Yeongtong-gu Suwon-si Gyeonggi-do443-710 (KR); Ha, Jun Yong, Gangnam-gu Seoul 135-940 (KR); Koh, Hyun Ju, Gunpo-si Kyunggi-do, 435-010 (KR); Moh, Joo Hyun, Gangnam-gu Seoul 135-544 (KR); Kim, Sung-Il, Yangju-si Gyeonggi-do 482-830 (KR); Park, Young-Ho, Dongjak-ku Seoul 156-775 (KR); Suh, Young-Ger, Sillim-dong Gwanak-gu Seoul 151-010 (KR); Kim, Hee-Doo Sookmyung Women's University, Youngsan-Ku Seoul 140-742 (KR); Park, Hyeung-Geun Seoul National University, Gwanak-gu Seoul 151-010 (KR); Oh, Uh Taek Seoul National University, Gwanak-gu Seoul 151-010 (KR)
(74) Representative: Blodig, Wolfgang

(57) **Abstract**

This present invention relates to novel compounds, of the formula (I), 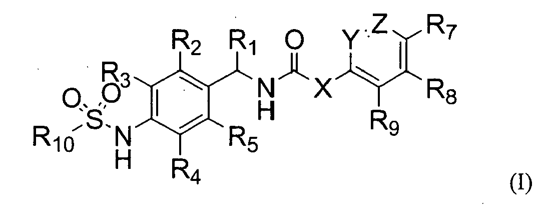 isomer thereof or pharmaceutically acceptable salts thereof as vanilloid receptor (Vanilloid Receptor 1;VR1;TRPV1) antagonist; and a pharmaceutical composition containing the same.
The present invention provides a pharmaceutical composition for preventing or treating a disease such as pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis and pancreatitis.

## Description

### Technical field

The present invention relates to novel compounds, isomer thereof or pharmaceutically acceptable salts thereof as TRPV1 antagonist; and a pharmaceutical composition containing the same

### Background Art

The vanilloid receptor-1 (VR1, or transient receptor potential vanilloid-1, TRPV1) is the receptor for capsaicin (8-methyl-N-vanillyl-6-nonenamide), a pungent ingredient in hot peppers. The molecular cloning of TRPV1 was reported in 1997 (Caterina et al., 1997, Nature, 389, pp816-824), which belongs to the TRP channel family of non-selective cation channel. TRPV 1 is activated or sensitized by stimuli such as capsaicin, resiniferatoxin, heat, acid, anandamide, lipid metabolites or the like ; thus it plays a crucial role as a molecular integrator of noxious stimuli in mammals (Tominaga et al., 1998, Neuron, 21 pp531-543; Hwang et al., 2000, PNAS, 97, pp6155-6160). The TRPV1 is highly expressed in primary afferent sensory neurons, and also reportedly expressed in various organs and tissues such as bladder, kidney, lung, intestine, or skin and in the central nervous system (CNS) including the brain and nonneuronal tissues (Mezey et al., 2000, PNAS, 97, pp3655-3660; Stander et al., 2004, Exp. Dermatol. 13, pp129-139; Cortright et al., 2001, BBRC, 281, pp1183-1189). Activation of the TRPV1 by endogenous/exogenous stimuli leads to not only transmission of noxious stimuli, but also liberation of neuropeptides such as substance P, CGRP (Calcitonin Gene-Related Peptide) in the neurons, thereby causing neurogenic inflammation.

TRPV1 knock-out mice showed normal responses in a wide range of behavioural tests including noxious mechanical and acute thermal stimuli, but exhibited little thermal hypersensitivity in inflammation states. (Caterina et- al., 2000, Science, 288, pp306-313; Davis et al., 2000, Nature, 405, pp183-187; Karai et al., 2004, J. Clin. Invest., 113, pp1344-1352).

As mentioned above, the TRPV1 knock-out mice exhibited reduced responses to thermal or noxious stimuli, which is thus raising the possibility that TRPV1 antagonists may be utilized for the prevention or treatment of various pain conditions. This possibility is supported by the report that the well-known TRPV1 antagonist, capsazepine, also decreases hyperalgesia caused by physical stimuli in several models of inflammatory and neuropathic pain (Walker et al., 2003, JPET, 304, pp56-62; Garcia-Martinez et al., 2002, PNAS, 99, 2374-2379). In addition, treatment of the primary culture of afferent sensory neurons with the TRPV1 agonist, capsaicin etc., results in damage to nerve functions and furthermore death of nerve cells. The TRPV1 antagonist exerts defense actions against such damage to nerve functions and nerve cell death (Holzer P, 1991, Pharmacological Reviews, 43, pp143-201; Mezey et al., 2000, PNAS, 97, 3655-3660). The TRPV1 is expressed on sensory neurons distributed in all regions of the gastrointestinal tract and is highly expressed in inflammatory disorders such as irritable bowel syndrome and inflammatory bowel disease (Chan et al., 2003, Lancet, 361, pp385-391 ; Yiangou et al., 2001, Lancet, 357, pp1338-1339). In addition, activation of the TRPV1 stimulates sensory nerves, which in turn causes release of neuropeptides which are known to play a critical role in pathogenesis of gastrointestinal disorders such as gastro-esophageal reflux disease (GERD) and stomach duodenal ulcer (Holzer P, 2004, Eur. J. Pharmacol. 500, pp231-241; Geppetti et al., 2004, Br. J. Pharmacol., 141, pp1313-1320).

The TRPV1-expressing afferent nerves are abundantly distributed in airway mucosa, and bronchial hypersensitivity is very similar mechanism to hyperalgesia. Protons and lipoxygenase products, known as endogenous ligands for the TRPV1, are well known as crucial factors responsible for development of asthma and chronic obstructive pulmonary diseases (Hwang et al., 2002, Curr. Opin. Pharmacol. pp235-242; Spina et al., 2002, Curr. Opin. Pharmacol. pp264-272). Further, it has been reported that air-polluting substances which are a kind of asthma-causing substances, i.e., particulate matter specifically acts on the TRPV1 and such action is inhibited by capsazepine (Veronesi et al., 2001, NeuroToxicology, 22, pp795-810). Urinary bladder hypersensitiveness and urinary incontinence are caused by various central/peripheral nerve disorders or injury, and capsaicin-responsive sensory nerves play an important role in bladder function control and inflammation. In addition, immunoreactivity of the TRPV1 was reported in urinary bladder epithelium (urothelium) in rats, and it was found that bladder overactivity induced by capsaicin was due to the stimulation of TRPV1 present in nerve fibers or various transmitters which are released by TRPV1 (Birder et al., 2001, PNAS, 98, pp13396-13401). Further, TRPV1 -/- mice are anatomically normal but have higher frequency of low-amplitude, non-voiding bladder contractions and reduced reflex voiding during bladder filling as compared to wild type mice, which is thus indicating that the TRPV1 affects functions of the bladder (Birder et al., 2002, Nat. Neuroscience, 5, pp856-860). The TFPV1 is distributed in human epidermal keratinocytes as well as in primary afferent sensory nerves (Denda et al., 2001, Biochem. Biophys. Res. Commun., 285, pp1250-1252; Inoue et al., 2002, Biochem. Biophys. Res. Commun., 291, pp124-129), and it is then involved in transmission of various noxious stimuli and pains such as skin irritation and pruritus, thereby having close correlation with etiology of dermatological diseases and disorders, such as skin inflammation, due to neurogenic/non-neurogenic factors. This is supported by the report that the TRPV1 antagonist, capsazepine inhibits inflammatory mediators in human skin cells (Southall et al., 2003, J. Pharmacol. Exp. Ther., 304, pp217-222).

Based on the above-mentioned information, development of various TRPV1 antagonists is under way, and some patents and patent applications relating to TRPV 1 antagonists under development were published. (Rami et al., 2004, Drug Discovery Today: Therapeutic Strategies, 1, pp97-104 ; Correll et al., 2006, Expert. Opin. Ther. Patents, 16, pp783-795 ; Kyle et al., 2006, Expert. Opin. Ther. Patents, 16, pp977-996)

Compounds of the present invention, are useful for prophylaxis and treatment of diseases associated with the activity of TRPV1 (Nagy et al., 2004, Eur. J. Pharmacol. 500, 351-369) including but not limited to, pain such as acute pain, chronic pain, neuropathic pain, post-operative pain, rheumatic arthritic pain, osteoarthritic pain, postherpetic neuralgia, neuralgia, headache, dental pain, pelvic pain and migraine (Petersen et al., 2000, Pain 88, pp125-133; Walker et al., 2003, J. Pharmacol. Exp. Ther., 304, pp56-62; Morgan et al.,2005, J. Orofac. Pain, 19, pp248-60 ; Dinis et al., 2005, Eur. Urol., 48, pp162-7; Akerman et al., 2004, Br. J. Pharmcol., 142, pp1354-1360) ; nerve-related diseases such as neuropathies, HIV-related neuropathy, nerve injury, neurodegeneration, and stroke (Park et al., 1999, Arch. Pharm. Res. 22, pp432-434; Kim et al., 2005, J. Neurosci. 25(3), pp662-671); diabetic neuropathy (Kamei et al., 2001, Eur. J. Pharmacol. 422, pp83-86); fecal urgency; irritable bowel syndrome (Chan et al., 2003, Lancet, 361, pp385-391); inflammatory bowel disease (Yiangou et al., 2001, Lancet 357, pp1338-1339); gastrointestinal disorders such as gastro-esophageal reflux disease (GERD), stomach duodenal ulcer and Crohn's disease (Holzer P, 2004, Eur. J. Pharm., 500, pp231-241; Geppetti et al., 2004, Br. J. Pharmacol., 141, pp1313-1320); respiratory diseases such as asthma, chronic obstructive pulmonary disease, cough (Hwang et al., 2002, Curr. Opin. Pharmacol. pp235-242; Spina et al., 2002, Curr. Opin. Pharmacol. pp264-272; Geppetti et al., 2006, Eur. J. Pharmacol., 533, pp207-214 ); urinary incontinence (Birder et al., 2002, Nat. Neuroscience 5, pp856-860); urinary bladder hypersensitiveness (Birder et al., 2001, PNAS, 98, pp13396-13401); neurotic/allergic/inflammatory skin diseases such as psoriasis, pruritus, prurigo and dermatitis (Southall et al., 2003, J. Pharmacol. Exp. Ther., 304, pp217-222); irritation of skin, eye or mucous membrane (Tominaga et al., 1998, Neuron 21 pp531-543); hyperacusis; tinnitus; vestibular hypersensitiveness (Balaban et al., 2003, Hear Res. 175, pp 165-70); cardiac diseases such as myocardial ischemia (Scotland et al., 2004, Circ. Res. 95, pp1027-1034; Pan et al., 2004, Circulation 110, pp1826-1831) ; hair growth-related disorders such as hirsutism, effluvium, alopecia (Bodó et al., 2005, Am. J. Patho. 166, pp985-998, Bíró et al., 2006, J. Invest. Dermatol. pp1-4) ; rhinitis (Seki et al. 2006, Rhinology, 44, pp128-34) ; pancreatitis (Hutter et al., 2005, Pancreas 30, pp260-5) ; cystitis (Dinis et al., 2004, J. Neurosci., 24, pp11253-63; Sculptoreanu et al., 2005, Neurosci. Lett. 381, pp42-6).

As a result of extensive and intensive studies based on the theoretical background discussed above, the present inventors have consequently synthesized novel compounds having VRI antagonistic activity by the replacement of one phenyl ring with a heterocyclic ring. Furthermore, the present inventors have also surprisingly identified that the replacement of one phenyl ring as mentioned above provided the improvement of their physicochemical characteristics, especially the solubility.

Therefore, it is an object of the present invention to provide novel compounds useful as a potent antagonist for a TRPV1, isomer thereof and pharmaceutically acceptable salts thereof; and a pharmaceutical composition comprising the same.

### Disclosure of the invention

The present invention provides a novel compound of the following formula (I), an isomer thereof, or a pharmaceutically acceptable salt thereof; and a pharmaceutical composition containing the same; 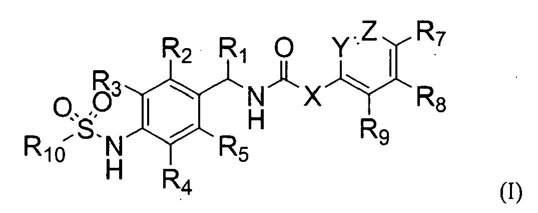 wherein,
X is CR₁₁=CR₁₂, CHR₁₁CHR₁₂, 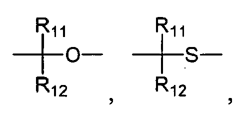 or C=C, wherein, R₁₁ and R₁₂, if present, are independently hydrogen, halogen, or alkyl preferably C1-C10 alkyl;
Y and Z are independently CH, CR₆, or N, such that at least one of Y and Z is N;
R₁ is hydrogen or alkyl, preferably C1-C10 alkyl;
R₂, R₃, R₄, and R₅ are independently hydrogen, halogen, nitro, cyano, alkyl (preferably C1-C10 alkyl), alkoxy (preferably C1-C10 alkoxy), haloalkyl (preferably halo (C1-C10) alkyl), alkenyl (preferably C2-C10 alkenyl), alkynyl (preferably C2-C10 alkynyl), carboxy, alkoxycarbonyl (preferably C1-C10 alkoxycarbonyl), or alkylthio (preferably C1-C10 alkylthio);
R₆, R₇, R₈, and R₉ are independently hydrogen, hydroxy, halogen, nitro, carboxy, alkyl (preferably C1-C10 alkyl), alkoxy (preferably C1-C10 alkoxy), hydroxyalkyl (preferably hydroxy (C1-C10) alkyl), alkenyl (preferably C2-C10 alkenyl), alkynyl (preferably C2-C 10 alkynyl), haloalkyl (preferably halo (C1-C10) alkyl), haloalkoxy (preferably halo (C1-C10) alkoxy), alkylthio (preferably C1-C10 alkylthio), alkylsufonyl (preferably C1-C10 alkylsulfonyl), alkylcarbonyl (preferably C1-C10 alkylcarbonyl), alkoxycarbonyl (preferably C1-C10 alkoxycarbonyl), alkenyloxy (preferably C2-C10 alkenyloxy), alkoxyalkoxy (preferably C1-C10 alkoxy (C1-C10) alkoxy), alkoxyalkoxyalkyl (preferably C1-C10 alkoxy (C1-C10) alkoxy (C1-C10) alkyl), alkylpiperazinyl (preferably C1-C10 alkylpiperazinyl), piperidyl, alkoxyalkylamino (preferably C1-C10 alkoxy (C1-C10) alkylamino), alkylamino (preferably C1-C10 alkylamino), dialkylamino (preferably di(C1-C10 alkyl)amino), cycloalkyl (preferably C3-C8 cycloalkyl) which may be unsubstituted or substituted with one or more alkyl groups preferably C1-C6 alkyl groups, pyrrolidinyl, phenyl, or morpholinyl, wherein the phenyl may be unsubstituted or substituted with one or more substituents selected from halogen, alkyl (preferably C1-C10 alkyl), and haloalkyl (preferably halo (C1-C10) alkyl); and
R₁₀ is alkyl (preferably C1-C10 alkyl), haloalkyl (preferably halo (C1-C10) alkyl), or alkynyl (preferably C2-C 10 alkenyl).

The present invention also provides a novel compound of the following formula (I), an isomer thereof, or a pharmaceutically acceptable salt thereof; and a pharmaceutical composition containing the same; 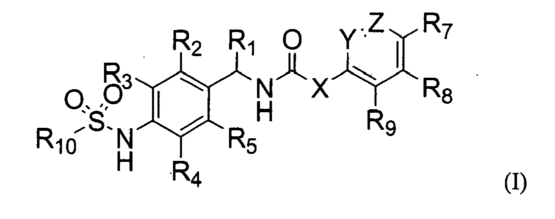 wherein,
X is CR₁₁=CR₁₂ , CHR₁₁CHR₁₂ , 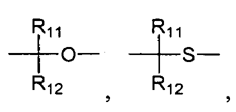 or C≡C, wherein, R₁₁ and R₁₂,if present, are independently hydrogen, halogen, or C1-C5 alkyl;
Y and Z are independently CH, CR₆, or N, such that at least one of Y and Z is N;
R₁ is hydrogen or C1-C5 alkyl;
R₂, R₃, R₄, and R₅ are independently hydrogen, halogen, nitro, cyano, C1-C5 alkyl, C1-C5 alkoxy, halo (C1-C5) alkyl, C2-C5 alkenyl, C2-C5 alkynyl, carboxy, C1-C5 alkoxycarbonyl, or C1-C5 alkylthio;
R₆, R₇, R₈, and R₉ are independently hydrogen, hydroxy, halogen, nitro, carboxy, C1-C5 alkyl, C1-C5 alkoxy, hydroxy (C1-C5) alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C5 alkyl)amino, C3-C6 cycloalkyl which may be unsubstituted or substituted with one or more C1-C3 alkyl groups, pyrrolidinyl, phenyl, or morpholinyl, wherein the phenyl may be unsubstituted or substituted with one or more substituents selected from halogen, C1-C5 alkyl, and halo (C1-C5) alkyl; and
R₁₀ is C1-C5 alkyl, halo (C1-C5) alkyl, or C2-C5 alkenyl.

Another aspect of the present invention is a compound according to the above formula (1), an isomer, or a pharmaceutically acceptable salt thereof;
wherein,
X is CR₁₁=CR₁₂, CHR₁₁CHR₁₂, 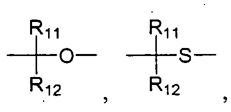 or C=C, wherein, R₁₁ and R12, if present, are independently hydrogen, halogen, or C1-C3 alkyl;
R₁ is hydrogen or C1-C3 alkyl;
R₂, R₃, R₄, and R₅ are independently hydrogen, halogen, nitro, cyano, methyl, ethyl, ethenyl, ethynyl, trifluoromethyl, methoxy, ethoxy, or methoxycarbonyl;
R₆, R₇, R₈, and R₉ are independently hydrogen, hydroxy, halogen, nitro, carboxy, C1-C5 alkyl, C1-C5 alkoxy, hydroxy (C1-C5) alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C3 alkyl)amino, C3-C6 cycloalkyl which may be unsubstituted or substituted with one or more methyl groups, pyrrolidinyl, phenyl, or morpholinyl, wherein the phenyl may be unsubstituted or substituted with one or more substituents selected from halogen, C1-C5 alkyl, and halo (C1-C5) alkyl; and
R₁₀ is C1-C3 alkyl or C2-C3 alkenyl.

The present invention also provides a novel compound of the following formula (II), an isomer, or a pharmaceutically acceptable salt thereof; 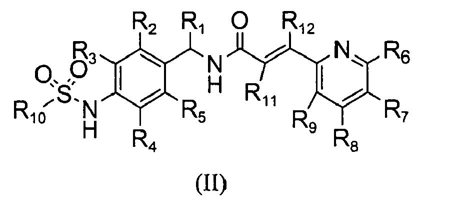 wherein,
R₁ is hydrogen or alkyl, preferably C1-C10 alkyl;
R₂, R₃, R₄, and R₅ are independently hydrogen, halogen, nitro, cyano, alkyl (preferably C1-C10 alkyl), alkoxy (preferably C1-C10 alkoxy), haloalkyl (preferably halo (C1-C10) alkyl), alkenyl (preferably C2-C10 alkenyl), alkynyl (preferably C2-C10 alkynyl), carboxy, alkoxycarbonyl (preferably C1-C10 alkoxycarbonyl), or alkylthio (preferably C1-C10 alkylthio);
R₆, R₇, R₈, and R₉ are independently hydrogen, hydroxy, halogen, nitro, carboxy, alkyl (preferably C1-C10 alkyl), alkoxy (preferably C1-C10 alkoxy), hydroxyalkyl (preferably hydroxy (C1-C10) alkyl), alkenyl (preferably C2-C 10 alkenyl), alkynyl (preferably C2-C10 alkynyl), haloalkyl (preferably halo (C1-C10) alkyl), haloalkoxy (preferably halo (C1-C10) alkoxy), alkylthio (preferably C1-C10 alkylthio), alkylsufonyl (preferably C1-C10 alkylsulfonyl), alkylcarbonyl (preferably C1-C10 alkylcarbonyl), alkoxycarbonyl (preferably C1-C10 alkoxycarbonyl), alkenyloxy (preferably C2-C10 alkenyloxy), alkoxyalkoxy (preferably C1-C10 alkoxy (C1-C10) alkoxy), alkoxyalkoxyalkyl (preferably C1-C10 alkoxy (C1-C10) alkoxy (C1-C10) alkyl), alkylpiperazinyl (preferably C1-C10 alkylpiperazinyl), piperidyl, alkoxyalkylamino (preferably C1-C10 alkoxy (C1-C10) alkylamino), alkylamino (preferably C1-C10 alkylamino), dialkylamino (preferably di(C1-C10 alkyl)amino), cycloalkyl (preferably C3-C8 cycloalkyl) which may be unsubstituted or substituted with one or more C1-C6 alkyl groups, pyrrolidinyl, phenyl, or morpholinyl, wherein the phenyl may be unsubstituted or substituted with one or more substituents selected from halogen, alkyl (preferably C1-C10 alkyl), and haloalkyl (preferably halo (C1-C10) alkyl); and
R₁₀ is alkyl (preferably C1-C10 alkyl), haloalkyl (preferably halo (C1-C10) alkyl), or alkynyl (preferably C2-C10 alkenyl).

The present invention also provides a novel compound of the following formula (II), an isomer, or a pharmaceutically acceptable salt thereof; 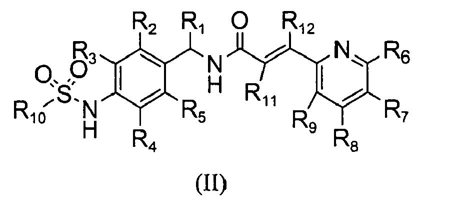 wherein,
R₁ is hydrogen or C1-C5 alkyl;
R₂, R₃, R₄, and R₅ are independently hydrogen, halogen, nitro, cyano, C1-C5 alkyl, C1-C5 alkoxy, halo (C1-C5) alkyl, C2-C5 alkenyl, C2-C5 alkynyl, carboxy, C1-C5 alkoxycarbonyl, or C1-C5 alkylthio;
R₆, R₇, R₈, and R₉ are independently hydrogen, hydroxy, halogen, nitro, carboxy, C1-C5 alkyl, C1-C5 alkoxy, C2-C5 alkenyl, C2-C5 alkynyl, halo (C1-C5) alkyl, halo (C1-C5) alkoxy, hydroxy (C1-C5) alkyl, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C5 alkyl)amino, C3-C6 cycloalkyl which may be unsubstituted or substituted with one or more C1-C3 alkyl groups, pyrrolidinyl, phenyl, or morpholinyl, wherein the phenyl may be unsubstituted or substituted with one or more substituents selected from halogen, C1-C5 alkyl, and halo (C1-C5) alkyl;
R₁₀ is C1-C5 alkyl, halo (C1-C5) alkyl, or C2-C5 alkenyl; and
R₁₁ and R₁₂ are independently hydrogen, C1-C5 alkyl, or halogen.

Also disclosed is a compound of the formula (III), an isomer, or a pharmaceutically acceptable salt thereof; 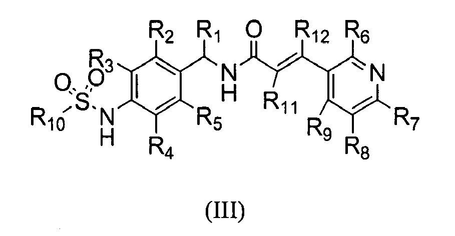 wherein,
R₁ is hydrogen or alkyl, preferably C1-C10 alkyl;
R₂, R₃, R₄, and R₅ are independently hydrogen, halogen, nitro, cyano, alkyl (preferably C1-C10 alkyl), alkoxy (preferably C1-C10 alkoxy), haloalkyl (preferably halo (C1-C10) alkyl), alkenyl (preferably C2-C10 alkenyl), alkynyl (preferably C2-C10 alkynyl), carboxy, alkoxycarbonyl (preferably C1-C10 alkoxycarbonyl), or alkylthio (preferably C1-C10 alkylthio);
R₆, R₇, R₈, and R₉ are independently hydrogen, hydroxy, halogen, nitro, carboxy, alkyl (preferably C1-C10 alkyl), alkoxy (preferably C1-C10 alkoxy), hydroxyalkyl (preferably hydroxy (C1-C10) alkyl), alkenyl (preferably C2-C10 alkenyl), alkynyl (preferably C2-C10 alkynyl), haloalkyl (preferably halo (C1-C10) alkyl), haloalkoxy (preferably halo (C1-C10) alkoxy), alkylthio (preferably C1-C10 alkylthio), alkylsufonyl (preferably C1-C10 alkylsulfonyl), alkylcarbonyl (preferably C1-C10 alkylcarbonyl), alkoxycarbonyl (preferably C1-C10 alkoxycarbonyl), alkenyloxy (preferably C2-C10 alkenyloxy), alkoxyalkoxy (preferably C1-C10 alkoxy (C1-C10) alkoxy), alkoxyalkoxyalkyl (preferably C1-C10 alkoxy (C1-C10) alkoxy (C1-C10) alkyl), alkylpiperazinyl (preferably C1-C10 alkylpiperazinyl), piperidyl, alkoxyalkylamino (preferably C1-C10 alkoxy (C1-C10) alkylamino), alkylamino (preferably C1-C10 alkylamino), dialkylamino (preferably di(C1-C10 alkyl)amino), cycloalkyl (preferably C3-C8 cycloalkyl) which may be unsubstituted or substituted with one or more C1-C6 alkyl groups, pyrrolidinyl, phenyl, or morpholinyl, wherein the phenyl may be unsubstituted or substituted with one or more substituents selected from halogen, alkyl (preferably C1-C10 alkyl), and haloalkyl (preferably halo (C1-C10) alkyl); and
R₁₀ is alkyl (preferably C1-C10 alkyl), haloalkyl (preferably halo (C1-C10) alkyl), or alkynyl (preferably C2-C 10 alkenyl).

One preferred aspect of the present invention is a compound of the formula (III), an isomer, or a pharmaceutically acceptable salt thereof; 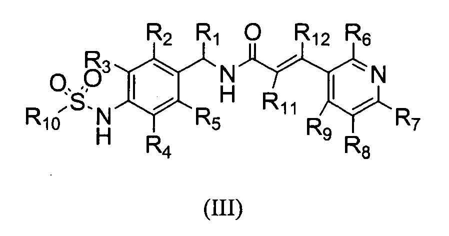 wherein,
R₁ is hydrogen or C1-C5 alkyl;
R₂, R₃, R₄, and R₅ are independently hydrogen, halogen, nitro, cyano, C1-C5 alkyl, C1-C5 alkoxy, halo (C1-C5) alkyl, C2-C5 alkenyl, C2-C5 alkynyl, carboxy, C1-C5 alkoxycarbonyl, or C1-C5 alkylthio;
R₆, R₇, R₈, and R₉ are independently hydrogen, hydroxy, halogen, nitro, carboxy, C1-C5 alkyl, C1-C5 alkoxy, C2-C5 alkenyl, C2-C5 alkynyl, halo (C1-C5) alkyl, halo (C1-C5) alkoxy, hydroxy (C1-C5) alkyl, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C5 alkyl)amino, C3-C6 cycloalkyl which may be unsubstituted or substituted with one or more C1-C3 alkyl groups, pyrrolidinyl, phenyl, or morpholinyl, wherein the phenyl may be unsubstituted or substituted with one or more substituents selected from halogen, C1-C5 alkyl, and halo (C1-C5) alkyl;
R₁₀ is C1-C5 alkyl, halo (C1-C5) alkyl, or C2-C5 alkenyl; and
R₁₁ and R₁₂ are independently hydrogen, C1-C5 alkyl, or halogen.

One aspect of the present invention is a compound of the formula (I), (II), or (III), an isomer, or a pharmaceutically acceptable salt thereof as defined further above, wherein R₇ is a C3-C6 cycloalkyl group which may be unsubstituted or substituted with one or more methyl groups, or a C1-C5 alkyl group that is optionally halogenated with one or more radicals selected from chloro, bromo, or, preferably, fluoro. Examples are methylcyclopropyl, CF₃, isopropyl, t-butyl and isobutyl.

Another aspect of the present invention is a compound of the formula (I), (II), or (III), an isomer, or a pharmaceutically acceptable salt thereof as defined further above, wherein R₆ is selected from hydrogen, fluoro, bromo, chloro, methyl, ethyl, hydroxymethyl, trifluoromethyl, morpholinyl, methoxy, and piperidyl.

Another aspect of the present invention is a compound of the formula (I), (II), or (III), an isomer, or a pharmaceutically acceptable salt thereof as defined further above, wherein R₃ is selected from hydrogen, fluoro, bromo, chloro, methyl, ethyl, trifluoromethyl, ethenyl, ethynyl, and cyano..

Another aspect of the present invention is a compound of the formula (I), (II), or (III), an isomer, or a pharmaceutically acceptable salt thereof as defined further above, wherein R₁₀ is methyl.

Another aspect of the present invention is a compound of the formula (I), (II), or (III), an isomer, or a pharmaceutically acceptable salt thereof as defined further above, wherein R₁₁ and R₁₂, if present, are both hydrogen.

Another aspect of the present invention is a compound of the formula (I), (II), or (III), an isomer, or a pharmaceutically acceptable salt thereof as defined further above, wherein R₄ is hydrogen, fluoro, chloro, bromo, methyl, ethyl, cyano, or trifluoromethyl.

Another aspect of the present invention is a compound of the formula (I), (II), or (III), an isomer, or a pharmaceutically acceptable salt thereof as defined further above, wherein R₁ and R₂ are both hydrogen.

Another aspect of the present invention is a compound of the formula (I), (II), or (III), an isomer, or a pharmaceutically acceptable salt thereof as defined further above, wherein R₁, R₂, and R₁₁ and R₁₂, if present, are all hydrogen.

Another aspect of the present invention is a compound of the formula (I), (II), or (III), an isomer, or a pharmaceutically acceptable salt thereof as defined further above, wherein R₁, R₂, and R₁₁ and R₁₂, if present, are all hydrogen, R₁₀ is methyl, and R₃ is selected from hydrogen, fluoro, bromo, chloro, methyl, ethyl, trifluoromethyl, ethenyl, ethynyl, and cyano.

Another aspect of the present invention is a compound of the formula (I), (II), or (III), an isomer, or a pharmaceutically acceptable salt thereof as defined further above, wherein R₁₁ and R₁₂, if present, are both hydrogen, R₁₀ is methyl or ethyl, R₃ is selected from hydrogen, fluoro, bromo, chloro, methyl, ethyl, trifluoromethyl, ethenyl, ethynyl, and cyano, and R₄ is selected from hydrogen, fluoro, chloro, bromo, methyl, cyano, or trifluoromethyl.

Another aspect of the present invention is a compound of the formula (I), (II), or (III), an isomer, or a pharmaceutically acceptable salt thereof as defined further above, wherein R₁₁ and R₁₂, if present, are both hydrogen, R₁₀ is methyl or ethyl, R₃ is selected from hydrogen, fluoro, bromo, chloro, methyl, ethyl, trifluoromethyl, ethenyl, ethynyl, and cyano, and R₇ is cyclopropylmethyl or a C1-C5 alkyl that is optionally halogenated with one or more radicals selected from chloro, bromo, or, preferably, fluoro.

Another aspect of the present invention is a compound of the formula (I), (II), or (III), an isomer, or a pharmaceutically acceptable salt thereof as defined further above, wherein R₁ and R₁₂, if present, are both hydrogen, R₁₀ is methyl or ethyl, R₂, R₅ and R₈ are hydrogen, R₃ is selected from hydrogen, fluoro, bromo, chloro, methyl, ethyl, trifluoromethyl, ethenyl, ethynyl, and cyano, and R₇ is a C1-C5 alkyl that is optionally halogenated with one or more radicals selected from chloro, bromo, or, preferably, fluoro.

One preferred aspect of the present invention is a compound of the formula (I), (II), or (III), an isomer, or a pharmaceutically acceptable salt thereof;
wherein,
R₁ is hydrogen, methyl, or ethyl;
R₂, R₃, R₄, and R₅ are independently hydrogen, fluoro, chloro, bromo, nitro, cyano, methyl, ethyl, ethenyl, ethynyl, trifluoromethyl, methoxy, ethoxy, or methoxycarbonyl;
R₆, if present, is hydrogen, hydroxy, fluoro, bromo, chloro, hydroxymethyl, C1-C5 alkyl, C1-C5 alkoxy, C2-C5 alkenyl, C2-C5 alkynyl, halo (C1-C5) alkyl, halo (C1-C5) alkoxy, C2-C5 alkenyloxy, C1-C5 alkoxy (C1-C5) alkoxy, C1-C5 alkoxy (C1-C5) alkoxy (C1-C5) alkyl, di(C1-C3 alkyl)amino, C1-C3 alkylpiperazinyl, piperidyl, pyrrolidinyl, halophenyl, phenyl, or morpholinyl;
R₇ is C1-C5 alkyl, halo (C1-C4) alkyl, halogen, piperidyl, morpholinyl, pyrrolidinyl, C3-C6 cycloalkyl which may be unsubstituted or substituted with one or more methyl groups, C2-C5 alkenyl;
R₈ and R₉ are independently hydrogen, halogen, or trifluoromethyl;
R₁₀ is C1-C5 alkyl, halo (C1-C5) alkyl, or C2-C5 alkenyl; and
R₁₁ and R₁₂, if present, are independently hydrogen, or methyl.

Another preferred embodiment of the invention is a compound of the formula (I), (II), or (III), an isomer, or a pharmaceutically acceptable salt thereof;
wherein,
R),and R₂ are hydrogen;
R₃ is hydrogen, fluoro, chloro, bromo, cyano, methyl, ethyl ethenyl, ethynyl, or trifluoromethyl;
R₄ and R₅ are independently hydrogen, fluoro, chloro, cyano, methyl, ethyl, or trifluoromethyl;
R₆, if present, is hydrogen, hydroxy, fluoro, bromo, chloro, methyl, hydroxymethyl, methoxy, trifluoromethyl, di(C1-C3 alkyl)amino, alkylpiperazinyl, piperidyl, pyrrolidinyl, halophenyl, phenyl, or morpholinyl;
R₇ is methyl, isopropyl, t-butyl, trifloromethyl, chloro, bromo, cyclopropyl which may be unsubstituted or substituted with one or two methyl groups, piperidyl, pyrrolidinyl, or morpholinyl;
R₈ is hydrogen;
R₉ is hydrogen or trifluoromethyl;
R₁₀ is C1-C5 alkyl, halo (C1-C5) alkyl, or C2-C5 alkenyl; and
R₁₁, and R₁₂, if present, are independently hydrogen or methyl.

Another preferred embodiment of the invention is a compound of the formula (I), (II), or (III), an isomer, or a pharmaceutically acceptable salt thereof;
wherein,
R₁, R₂, and R₅ are hydrogen;
R₃ is hydrogen, fluoro, chloro, cyano, methyl, ethenyl, ethynyl, or trifluoromethyl;
R₄ is hydrogen, fluoro, chloro, cyano, methyl, ethyl, or trifluoromethyl;
R₆, if present, is hydrogen, hydroxy, fluoro, bromo, chloro, methyl, hydroxymethyl, methoxy, trifluoromethyl, diethylamino, piperidyl, pyrrolidinyl, trifluorophenyl, phenyl, or morpholinyl;
R₇ is methyl, isopropyl, t-butyl, trifluoromethyl, chloro, bromo, cyclopropyl, methylcyclopropyl, piperidyl, pyrrolidinyl, or morpholinyl;
R₈ is hydrogen;
R₁₁, and R₁₂, if present, are hydrogen;
R₉ is hydrogen or trifluoromethyl; and
R₁₀ is methyl.

Another preferred embodiment of the invention is a compound of the formula (I), (II), or (III), an isomer, or a pharmaceutically acceptable salt thereof;
wherein,
R₁ is hydrogen or methyl;
R₂ is hydrogen;
R₃ is hydrogen, fluoro, chloro, bromo, cyano, methyl, ethyl, ethenyl, ethynyl, or trifluoromethyl;
R₄ and R₅ are independently hydrogen, fluoro, chloro, cyano, methyl, ethyl, or trifluoromethyl;
R₆, if present, is hydrogen, fluoro, chloro, bromo, methyl, methoxy, piperidyl, or morpholinyl;
R₇ is isopropyl, t-butyl, or trifluoromethyl;
R₈ is hydrogen;
R₁₁, and R₁₂, if present, are hydrogen;
R₉ is hydrogen or trifluoromethyl; and
R₁₀ is methyl.

Preferred examples of compounds according to the invention are selected from the group consisting of;
3-(6-tert-Butyl-pyridin-3-yl)-N-(3-chloro-4-methanesulfonylamino-benzyl)-acrylamide,
3-(6-tert-Butyl-pyridin-3-yl)-N-(3-fluoro-4-methanesulfonylamino-benzyl)-acrylamide,
3-(6-tert-Butyl-pyridin-3-yl)-N-(3-ethynyl-5-fluoro-4-methanesulfonylamino-benzyl)-acrylamide,
3-(6-tert-Butyl-pyridin-3-yl)-N-(4-methanesulfonylamino-3-vinyl-benzyl)-acrylamide,
N-(3-Fluoro-4-methanesulfonylamino-benzyl)-3-(2-morpholin-4-yl-6-trifluoromethylpyridin-3-yl)-acrylamide,
N-(4-Methanesulfonylamino-3-vinyl-benzyl)-3-(2-morpholin-4-yl-6-trifluoromethylpyridin-3-yl)-acrylamide,
N-(3-Chloro-4-methanesulfonylamino-benzyl)-3-(2-morpholin-4-yl-6-trifluoromethylpyridin-3-yl)-acrylamide,
N-(3-Fluoro-4-methanesulfonylamino-5-methyl-benzyl)-3-(2-morpholin-4-yl-6-trifluoromethyl-pyridin-3-yl)-acrylamide,
3-(6-tert-Butyl-2-methoxy-pyridin-3-yl)-N-(3-fluoro-4-methanesulfonylamino-benzyl)-acrylamide,
3-(6-tert-Butyl-pyridin-3-yl)-N-(3-fluoro-4-methanesulfonylamino-5-vinyl-benzyl)-acrylamide,
3-(6-tert-Butyl-2-methoxy-pyridin-3-yl)-N-(3-ethynyl-5-fluoro-4-methanesulfonylamino-benzyl)-acrylamide,
N-(3-Ethynyl-5-fluoro-4-methanesulfonylamino-benzyl)-3-(2-morpholin-4-yl-6-trifluoromethyl-pyridin-3-yl)-acrylamide,
3-(6-tert-Butyl-4-trifluoromethyl-pyridin-3-yl)-N-(3-fluoro-4-methanesulfonylamino-benzyl)-acrylamide,
3-(6-tert-Butyl-4-trifluoromethyl-pyridin-3-yl)-N-(3-ethynyl-5-fluoro-4-methanesulfonylamino-benzyl)-acrylamide,
N-(3,5-Difluoro-4-methanesulfonylamino-benzyl)-3-(2-morpholin-4-yl-6-trifluoromethyl-pyridin-3-yl)-acrylamide,
N-(3-Ethynyl-5-fluoro-4-methanesulfonylamino-benzyl)-3-(6'-trifluoromethyl-3,4,5,6-tetrahydro-2H-[1,2']bipyridinyl-3'-yl)-acrylamide,
3-(2-Bromo-6-tert-butyl-pyridin-3-yl)-N-(3-fluoro-4-methanesulfonylamino-benzyl)-acrylamide,
3-(2-Bromo-6-tert-butyl-pyridin-3-yl)-N-(3-ethynyl-5-fluoro-4-methanesulfonylamino-benzyl)-acrylamide, and
N-(3-Fluoro-4-methanesulfonylamino-benzyl)-3-(6'-trifluoromethyl-3,4,5,6-tetrahydro-2H-[1,2']bipyridinyl-3'-yl)-acrylamide.

Particularly preferred compounds according to the present invention are
3-(6-tert-Butyl-pyridin-3-yl)-N-(3-ethynyl-5-fluoro-4-methanesulfonylamino-benzyl)-acrylamide,
3-(6-tert-Butyl-pyridin-3-yl)-N-(4-methanesulfonylamino-3-vinyl-benzyl)-acrylamide,
N-(4-Methanesulfonylamino-3-vinyl-benzyl)-3-(2-morpholin-4-yl-6-trifluoromethyl-pyridin-3-yl)-acrylamide,
N-(3-Fluoro-4-methanesulfonylamino-5-methyl-benzyl)-3-(2-morpholin-4-yl-6-trifluoromethyl-pyridin-3-yl)-acrylamide,
3-(6-tert-Butyl-2-methoxy-pyridin-3-yl)-N-(3-fluoro-4-methanesulfonylamino-benzyl)-acrylamide,
3-(6-tert-Butyl-pyridin-3-yl)-N-(3-fluoro-4-methanesulfonylamino-5-vinyl-benzyl)-acrylamide,
3-(6-tert-Butyl-2-methoxy-pyridin-3-yl)-N-(3-ethynyl-5-fluoro-4-methanesulfonylamino-benzyl)-acrylamide,
N-(3-Ethynyl-5-fluoro-4-methanesulfonylamino-benzyl)-3-(2-morpholin-4-yl-6-trifluoromethyl-pyridin-3-yl)-acrylamide,
3-(6-tert-Butyl-4-trifluoromethyl-pyridin-3-yl)-N-(3-fluoro-4-methanesulfonylamino-benzyl)-acrylamide,
3-(6-tert-Butyl-4-trifluoromethyl-pyridin-3-yl)-N-(3-ethynyl-5-fluoro-4-methanesulfonylamino-benzyl)-acrylamide,
N-(3-Ethynyl-5-fluoro-4-methanesulfonylamino-benzyl)-3-(6'-trifluoromethyl-3,4,5,6-tetrahydro-2H-[1,2']bipyridinyl-3'-yl)-acrylamide,
3-(2-Bromo-6-tert-butyl-pyridin-3-yl)-N-(3-fluoro-4-methanesulfonylamino-benzyl)-acrylamide, and
3-(2-Bromo-6-tert-butyl-pyridin-3-yl)-N-(3-ethynyl-5-fluoro-4-methanesulfonylamino-benzyl)-acrylamide.

The present invention also provides a novel compound of the following formula (IV), an isomer, or a pharmaceutically acceptable salt thereof; 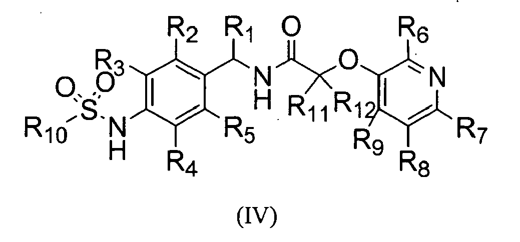 wherein,
R₁ is hydrogen or alkyl, preferably C1-C10 alkyl;
R₂, R₃, R₄, and R₅ are independently hydrogen, halogen, nitro, cyano, alkyl preferably C1-C10 alkyl, alkoxy preferably C1-C10 alkoxy, haloalkyl preferably halo (C1-C10) alkyl, alkenyl preferably C2-C10 alkenyl, alkynyl preferably C2-C10 alkynyl, carboxy, alkoxycarbonyl preferably C1-C10 alkoxycarbonyl, or alkylthio, preferably C1-C10 alkylthio;
R₆, R₇, R₈, and R₉ are independently hydrogen, hydroxy, halogen, nitro, carboxy, alkyl preferably C1-C10 alkyl, alkoxy preferably C1-C10 alkoxy, hydroxyalkyl preferably hydroxy (C1-C10) alkyl, alkenyl preferably C2-C10 alkenyl, alkynyl preferably C2-C10 alkynyl, haloalkyl preferably halo (C1-C10) alkyl, haloalkoxy preferably halo (C1-C10) alkoxy, alkylthio preferably C1-C10 alkylthio, alkylsufonyl preferably C1-C10 alkylsulfonyl, alkylcarbonyl preferably C1-C10 alkylcarbonyl, alkoxycarbonyl preferably C1-C10 alkoxycarbonyl, alkenyloxy preferably C2-C10 alkenyloxy, alkoxyalkoxy preferably C1-C10 alkoxy (C1-C10) alkoxy, alkoxyalkoxyalkyl preferably C1-C10 alkoxy (C1-C10) alkoxy (C1-C10) alkyl, alkylpiperazinyl preferably C1-C10 alkylpiperazinyl, piperidyl, alkoxyalkylamino preferably C1-C10 alkoxy (C1-C10) alkylamino, alkylamino preferably C1-C10 alkylamino, dialkylamino preferably di(C1-C10 alkyl)amino, cycloalkyl preferably C3-C8 cycloalkyl which may be unsubstituted or substituted with one or more C1-C6 alkyl groups, pyrrolidinyl, phenyl, or morpholinyl, wherein the phenyl may be unsubstituted or substituted with one or more substituents selected from halogen, alkyl preferably C1-C10 alkyl, and haloalkyl preferably halo (C1-C10) alkyl; and
R₁₀ is alkyl preferably C1-C10 alkyl, haloalkyl preferably halo (C1-C10) alkyl, or alkynyl preferably C2-C10 alkenyl.

The present invention also provides a novel compound of the following formula (IV), an isomer, or a pharmaceutically acceptable salt thereof; 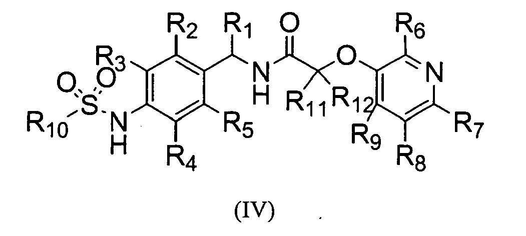 wherein,
R₁ is hydrogen, methyl, or ethyl;
R₂, R₃, R₄, and R₅ are independently hydrogen, fluoro, chloro, bromo, nitro, cyano, methyl, ethyl, ethenyl, ethynyl, trifluoromethyl, methoxy, ethoxy, hydroxy, or methoxycarbonyl;
R₆ is hydrogen, fluoro, bromo, chloro, hydroxymethyl, C1-C5 alkyl, C1-C5 alkoxy, C2-C5 alkenyl, C2-C5 alkynyl, halo (C1-C5) alkyl, halo (C1-C5) alkoxy, C2-C5 alkenyloxy, C1-C5 alkoxy (C1-C5) alkoxy, piperidyl, or morpholinyl;
R₇ is C1-C5 alkyl, halo (C1-C4) alkyl, halogen, morpholinyl, C3-C6 cycloalkyl, C2-C5 alkenyl, or nitro;
R₈ and R₉ are hydrogen;
R₁₀ is C1-C5 alkyl, halo (C1-C5) alkyl, or C2-C5 alkenyl; and
R₁₁ and R₁₂ are independently hydrogen, halogen, or methyl.

One preferred aspect of the present invention is a compound of above formula (IV), an isomer, or a pharmaceutically acceptable salt thereof;
wherein,
R₁, R₂, R₅, R₈, and R₉ are hydrogen;
R₃ and R₄ are independently hydrogen, fluoro, chloro, cyano, methyl, ethenyl, ethynyl, or trifluoromethyl;
R₆ is hydrogen, fluoro, bromo, chloro, hydroxymethyl, methoxy, morpholinyl, or trifluoromethyl;
R₇ is t-butyl, trifloromethyl, methyl, chloro, bromo, nitro, or morpholinyl; and
R₁₀ is methyl or ethenyl.

Another preferred aspect of the present invention is a compound of above formula (IV), an isomer, or a pharmaceutically acceptable salt thereof;
wherein,
R₁, R₂, R₅, R₈, and R₉ are hydrogen;
R₃ and R₄ are independently hydrogen, fluoro, chloro, cyano, methyl, ethenyl, ethynyl, or trifluoromethyl;
R₆ is hydrogen, fluoro, bromo, chloro, hydroxymethyl, methoxy, morpholinyl, or trifluoromethyl;
R₇ is t-butyl, trifloromethyl, methyl, chloro, bromo, nitro, or morpholinyl; and
R₁₀ is methyl.

The present invention also provides a novel compound of the following formula (V), an isomer, or a pharmaceutically acceptable salt thereof; 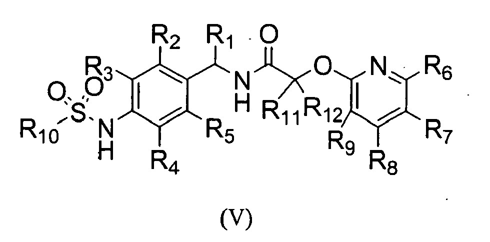 wherein,
R₁ is hydrogen or alkyl, preferably C1-C10 alkyl;
R₂, R₃, R₄, and R₅ are independently hydrogen, halogen, nitro, cyano, alkyl preferably C1-C10 alkyl, alkoxy preferably C1-C10 alkoxy, haloalkyl preferably halo (C1-C10) alkyl, alkenyl preferably C2-C10 alkenyl, alkynyl preferably C2-C10 alkynyl, carboxy, alkoxycarbonyl preferably C1-C10 alkoxycarbonyl, or alkylthio, preferably C1-C10 alkylthio;
R₆, R₇, R₈, and R₉ are independently hydrogen, hydroxy, halogen, nitro, carboxy, alkyl preferably C1-C10 alkyl, alkoxy preferably C1-C10 alkoxy, hydroxyalkyl preferably hydroxy (C1-C10) alkyl, alkenyl preferably C2-C 10 alkenyl, alkynyl preferably C2-C 10 alkynyl, haloalkyl preferably halo (C1-C10) alkyl, haloalkoxy preferably halo (C1-C10) alkoxy, alkylthio preferably C1-C10 alkylthio, alkylsufonyl preferably C1-C10 alkylsulfonyl, alkylcarbonyl preferably C1-C10 alkylcarbonyl, alkoxycarbonyl preferably C1-C10 alkoxycarbonyl, alkenyloxy preferably C2-C10 alkenyloxy, alkoxyalkoxy preferably C1-C10 alkoxy (C1-C10) alkoxy, alkoxyalkoxyalkyl preferably C1-C10 alkoxy (C1-C10) alkoxy (C1-C10) alkyl, alkylpiperazinyl preferably C1-C10 alkylpiperazinyl, piperidyl, alkoxyalkylamino preferably C1-C10 alkoxy (C1-C10) alkylamino, alkylamino preferably C1-C10 alkylamino, dialkylamino preferably di(C1-C10 alkyl)amino, cycloalkyl preferably C3-C8 cycloalkyl which may be unsubstituted or substituted with one or more C1-C6 alkyl groups, pyrrolidinyl, phenyl, or morpholinyl, wherein the phenyl may be unsubstituted or substituted with one or more substituents selected from halogen, alkyl preferably C1-C10 alkyl; and haloalkyl preferably halo (C1-C10) alkyl; and
R₁₀ is alkyl preferably C1-C10 alkyl, haloalkyl preferably halo (C1-C10) alkyl, or alkynyl preferably C2-C10 alkenyl.

The present invention also provides a novel compound of the following formula (V), an isomer, or a pharmaceutically acceptable salt thereof; 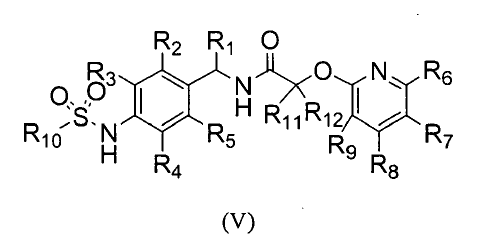 wherein,
R₁ is hydrogen, methyl, or ethyl;
R₂, R₃, R₄, and R₅ are independently hydrogen, fluoro, chloro, bromo, nitro, cyano, methyl, ethyl, ethenyl, ethynyl, trifluoromethyl, methoxy, ethoxy, or methoxycarbonyl;
R₆, R₇, R₈ and R₉ are independently hydrogen, fluoro, bromo, chloro, hydroxymethyl, C1-C5 alkyl, C1-C5 alkoxy, C2-C5 alkenyl, C2-C5 alkynyl, halo (C1-C5) alkyl, halo (C1-C5) alkoxy, C2-C5 alkenyloxy, C1-C5 alkoxy (C1-C5) alkoxy, piperidyl, nitro, or morpholinyl;
R₁₀ is C1-C5 alkyl, halo (C1-C5) alkyl, or C2-C5 alkenyl; and
R₁₁ and R₁₂ are independently hydrogen, halogen, or methyl.

One preferred aspect of the present invention is a compound of above formula (V), an isomer, or a pharmaceutically acceptable salt thereof;
wherein,
R₁, R₂, R₅, R₆, R₈, and R₉ are hydrogen;
R₃ and R₄ are independently hydrogen, fluoro, chloro, cyano, methyl, ethenyl, ethynyl, or trifluoromethyl;
R₇ is t-butyl, trifloromethyl, methyl, chloro, bromo, nitro, or morpholinyl; and
R₁₀ is methyl or ethenyl.

Another preferred aspect of the present invention is a compound of above formula (V), an isomer, or a pharmaceutically acceptable salt thereof;
wherein,
R₁, R₂, R₅, R₆, R₈, and R₉ are hydrogen;
R₃ and R₄ are independently hydrogen, fluoro, chloro, cyano, methyl, ethenyl, ethynyl, or trifluoromethyl;
R₇ is t-butyl, trifluoromethyl, methyl, chloro, bromo, nitro, or morpholinyl; and
R₁₀ is methyl.

The compounds of the formula (I), (II), (III), (IV), and (V) of the present invention can chemically be synthesized by the following reaction schemes. However, these are given only for illustration of the invention and not intended to limit to them.

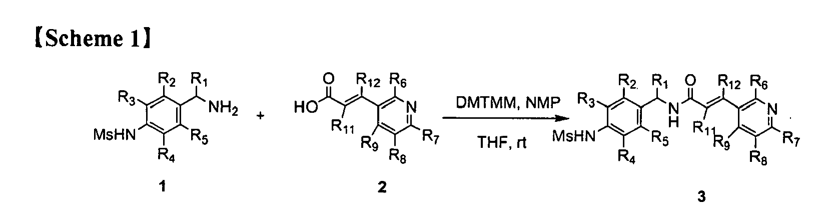

The Scheme 1 shows a proposed process for synthesizing acrylamide compound with various substituents. Substituted benzylamine (1) is reacted with pyridinylacrylic acid (2) to yield benzyl pyridinylacrylamide (3) using DMTMM {4-(4,6-dimethoxy-1,3,5-triazin-2-yl)-4-methylmorpholinium chloride} (Tetrahedron Lett., 1999, 40, 5327).

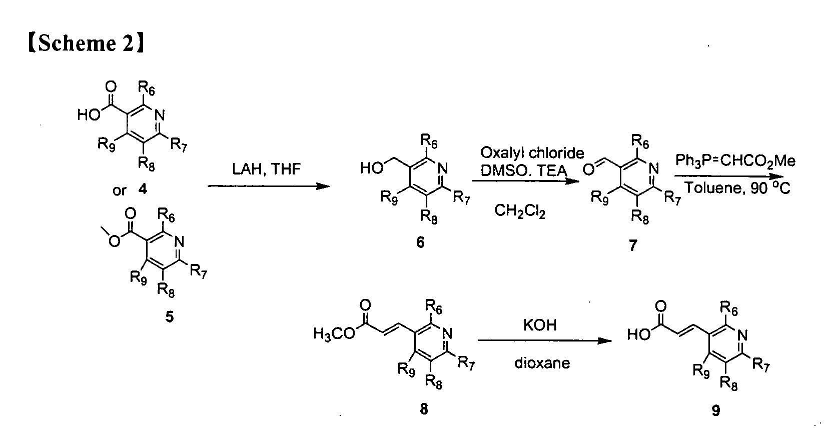

The Scheme 2 shows a proposed process for synthesizing pyridinyl acrylic acid (9) with various substituents. Substituted pyridinecarboxaldehyde (7) is prepared by known methods. Substituted nicotinic acid (4) and nicotinic ester (5) are converted to corresponding pyridinecarboxaldehyde (7) via pyridinyl methyl alcohol (6). Pyridinyl methanol is converted to pyridinylcarboxaldehyde (7) via swern oxidation. Pyridinecarboxaldehye (7) is converted to methyl pyridinyl acrylic ester (8) by the Wittig reaction. Methyl pyridinyl acrylic ester (8) is hydrolyzed with potassium hydroxide to yield pyridinyl acrylic acid (9).

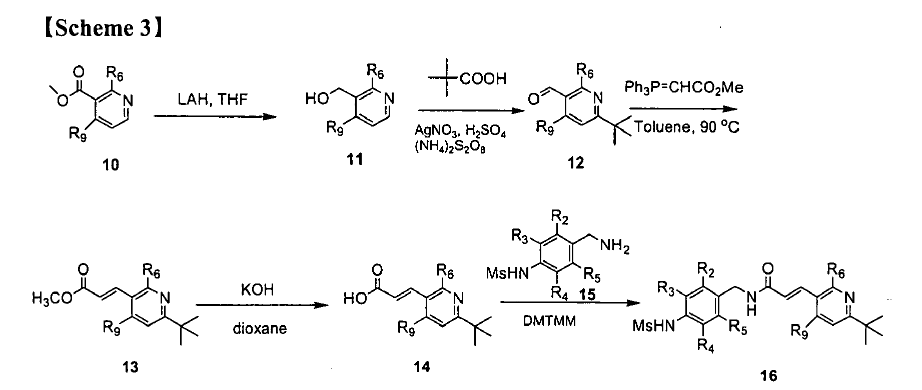

The Scheme 3 shows a proposed process for synthesizing pyridinyl acrylic amide (**16**) with t-butyl group. Substituted pyridinecarboxaldehyde with t-butyl group (**12**) is prepared by reduction and radical substitution method (J. Heterocyclic Chem., 1989, 25, 45-48). Substituted nicotinic ester (**10**) is converted to corresponding pyridine-3-methanol (**11**). Pyridinyl-3-methanol is reacted with pivalic acid and silver nitrate to give pyridinylcarboxaldehyde (**12**) *via* Tada's radical substitution. Compound **14** is synthesized from compound **12** with similar method of scheme 2. 4-t-Butylpyridinyl acrylic acid (**14**) is reacted with compound **15** to yield compound **16.**

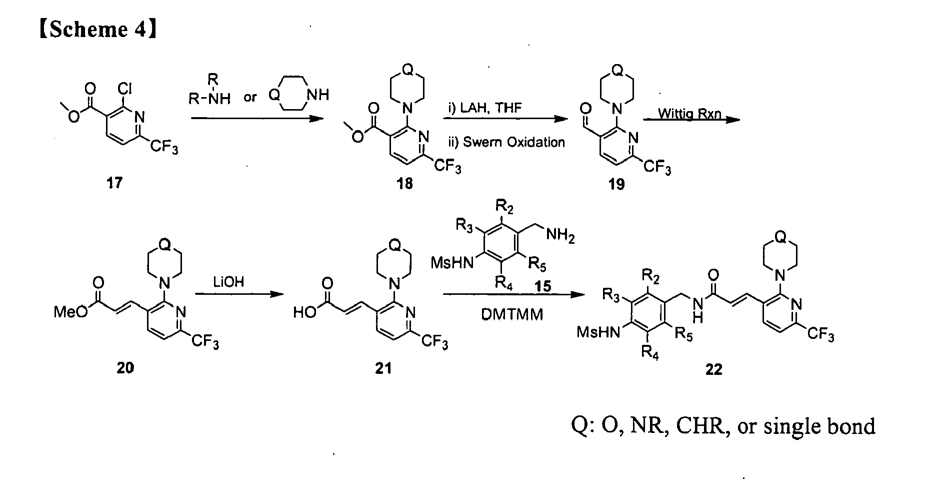

The Scheme 4 shows a proposed process for synthesizing pyridinyl acrylamide (22) with trifluoromethyl group. 2-chloro-6-trifluoromethyl-nicotinic ester (17) was reacted with cyclic secondary amine to give compound (18). Compound (18) was converted to obtain compound (21) under similar reaction of scheme 2. Compound (21) was reacted with compound (15) to give compound (22). 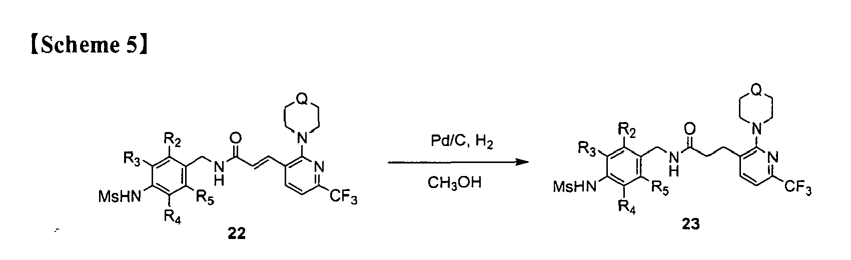 The Scheme 5 shows a proposed process for synthesizing pyridinyl propionamide (**23**) with trifluoromethyl group. Compound (**22**) is transformed to give compound (**23**) under hydrogen atmosphere. 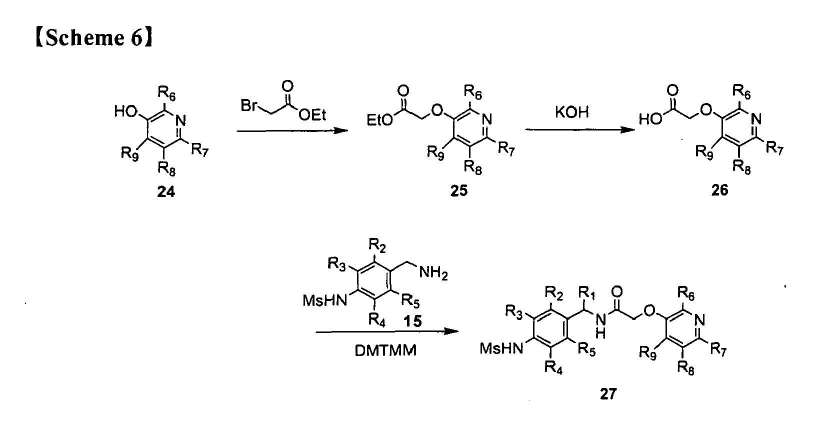 The Scheme 6 shows a proposed process for synthesizing pyridinoxy ethylamide (**27**). 3-hydroxy-pyridine (**24**) is reacted with ethyl bromoacetate to give compound (**25**). The compound (**25**) is hydrolyzed with potassium hydroxide to give 3-pyridinyloxyacetic acid (**26**). Compound (**26**) is reacted with compound (**15**) to yield compound (**27**).

The present invention also provides a compound of formula (I), (V), (III), (IV) or (V), an isomer thereof, or a pharmaceutically acceptable salt thereof for preventing or treating a disease associated with the pathological stimulation and/or aberrant expression of vanilloid receptor, wherein said composition comprises the compound of formula (I), (II), (III), (IV) or (V), an isomer thereof or a pharmaceutically acceptable salt thereof; and pharmaceutically acceptable carrier.

In one preferred aspect, the present invention provides a compound of formula (I), (V), (III), (IV) or (V), an isomer thereof, or a pharmaceutically acceptable salt thereof for treating a condition selected from the group consisting of pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis and pancreatitis.

In a particularly preferred aspect, the present invention relates to a compound of formula (I), (V), (III), (IV) or (V), an isomer thereof, or a pharmaceutically acceptable salt thereof, wherein the pain is or is associated with a condition selected from the group consisting of osteoarthritis, rheumatoid arthritis, ankylosing spondylitis, diabetic neuropathic pain, post-operative pain, dental pain, non-inflammatory musculoskeletal pain (including fibromyalgia, myofascial pain syndrome and back pain), migraine and other types of headaches.

The present invention also provides a pharmaceutical composition comprising a compound of formula (I), (V), (III), (IV) or (V), an isomer thereof, or a pharmaceutically acceptable salt thereof as an active ingredient together with a pharmaceutically acceptable carrier.

The present invention also provides a pharmaceutical composition for preventing or treating a disease associated with the pathological stimulation and/or aberrant expression of vanilloid receptor, wherein said composition comprises the compound of formula (I), (II), (III), (IV) or (V), an isomer thereof or a pharmaceutically acceptable salt thereof; and pharmaceutically acceptable carrier.

In one preferred aspect, the present invention provides a pharmaceutical composition comprising a compound of formula (I), (V), (III), (IV) or (V), an isomer thereof, or a pharmaceutically acceptable salt thereof, for treating a condition selected from the group consisting of pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis and pancreatitis.

In a particularly preferred aspect, the present invention relates to the pharmaceutical composition comprising a compound of formula (I), (V), (III), (IV) or (V), an isomer thereof, or a pharmaceutically acceptable salt thereof for treating pain as described above, wherein the pain is or is associated with a condition selected from the group consisting of osteoarthritis, rheumatoid arthritis, ankylosing spondylitis, diabetic neuropathic pain, post-operative pain, dental pain, non-inflammatory musculoskeletal pain (including fibromyalgia, myofascial pain syndrome and back pain), migraine and other types of headaches.

The present invention also provides a pharmaceutical composition comprising a compound of formula (I), (V), (III), (IV) or (V), an isomer thereof, or a pharmaceutically acceptable salt thereof, which is characterized in that it is adapted for oral administration.

In another aspect, the present invention relates to a method for inhibiting vanilloid ligand from binding to vanilloid receptor in a patient, comprising contacting cells expressing vanilloid receptor in the patient with the compound of formula (I), (II), (III), (IV) or (V), an isomer thereof, or a pharmaceutically acceptable salt thereof.

In another aspect, the present invention also provides a method for preventing or treating a condition selected from the group consisting of pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis and pancreatitis, which comprises administering to a mammal including a person in need thereof a therapeutically effective amount of the compound of formula (I), (II), (III), (IV), or (V), an isomer thereof, or a pharmaceutically acceptable salt thereof.

In a particularly preferred aspect, the present invention relates to the method of treating pain by administering a compound of formula (I), (V), (III), (IV) or (V), an isomer thereof, or a pharmaceutically acceptable salt thereof as described above, wherein the pain is or is associated with a condition selected from the group consisting of osteoarthritis, rheumatoid arthritis, ankylosing spondylitis, diabetic neuropathic pain, post-operative pain, dental pain, non-inflammatory musculoskeletal pain (including fibromyalgia, myofascial pain syndrome and back pain), migraine, and other types of headaches.

In another aspect, the present invention relates to the use of a compound of formula (I), (II), (III), (IV) or (V), an isomer thereof, or a pharmaceutically acceptable salt thereof for the preparation of a medicament for the prevention or treatment of a condition that is associated with the aberrant expression and/or aberrant activation of a vanilloid receptor.

In another aspect, the present invention relates to the use of a compound of formula (I), (II), (III), (IV) or (V), an isomer thereof, or a pharmaceutically acceptable salt thereof, in preparation of a medicament for the prevention or treatment of a condition that is selected from the group consisting of pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis and pancreatitis.

In a particularly preferred aspect, the present invention relates to the use of the compound of formula (I), (II), (III), (IV) or (V) for preparing a medicament for preventing or treating pain as described above, wherein the condition is pain or which is or which is associated with a condition selected from the group consisting of osteoarthritis, rheumatoid arthritis, ankylosing spondylitis, diabetic neuropathic pain, post-operative pain, dental pain, non-inflammatory musculoskeletal pain (including fibromyalgia, myofascial pain syndrome and back pain), migraine, and other types of headaches.

The present invention also provides a process for preparing a compound represented by the formula (III) 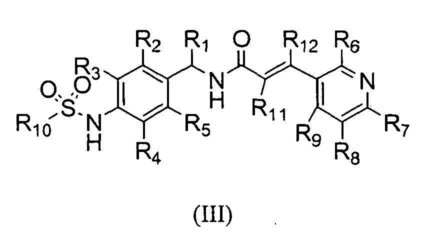 which comprises reacting a compound represented by the formula (IIIa); 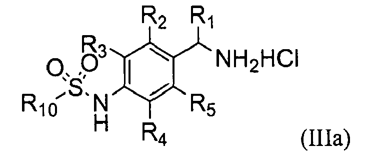 with a compound represented by the formula (IIIb); 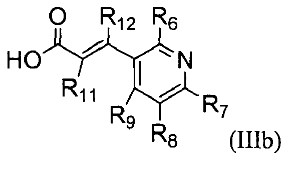 wherein,
R₁ is hydrogen or C1-C5 alkyl;
R₂, R₃, R₄, and R₅ are independently hydrogen, halogen, nitro, cyano, C1-C5 alkyl, C1-C5 alkoxy, halo (C1-C5) alkyl, C2-C5 alkenyl, C2-C5 alkynyl, carboxy, C1-C5 alkoxycarbonyl, or C1-C5 alkylthio;
R₆, R₇, R₈, and R₉ are independently hydrogen, hydroxy, halogen, nitro, carboxy, C1-C5 alkyl, C1-C5 alkoxy, C2-C5 alkenyl, C2-C5 alkynyl, halo (C1-C5) alkyl, halo (C1-C5) alkoxy, hydroxy (C1-C5) alkyl, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C5 alkyl)amino, C3-C6 cycloalkyl which may be unsubstituted or substituted with one or more C1-C3 alkyl groups, pyrrolidinyl, phenyl, or morpholinyl, wherein the phenyl may be unsubstituted or substituted with one or more substituents selected from halogen, C1-C5 alkyl, and halo (C1-C5) alkyl;
Rio is C1-C5 alkyl, halo (C1-C5) alkyl, or C2-C5 alkenyl; and
R₁₁ and R₁₂ are independently hydrogen, C1-C5 alkyl, or halogen.

One preferred aspect of the present invention is the process for preparing a compound of formula (III), wherein, the reaction is conducted in the presence of a coupling agent.

Another preferred aspect of the present invention is the process for preparing a compound of formula (III), wherein the coupling agent is selected from the group consisting of DCC (N,N-dicyclohexylcarbodidimide), EDCI {1-(3-dimethylaminopropyl)-3-ethylcarbodidimide hydrochloride (EDCI)}, and DMTMM {4-(4,6-dimethoxy-1,3,5-triazin-2-yl)-4-methylmorpholinium chloride}.

The present invention also provides a process for preparing a compound of formula (IIIc), 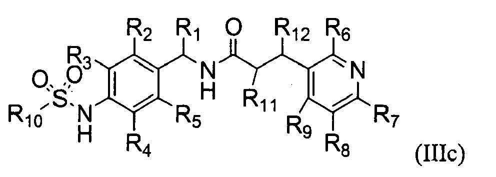 which comprises an reducing step of the compound of the formula (III).

One preferred aspect of the present invention is the process for preparing a compound of formula (IIIc), wherein the reducing step is conducted in the presence of hydrogen gas and palladium on carbon.

Another preferred aspect of the present invention is the process for preparing a compound of formula (III) or (IIIc), wherein R₁, R₁₁, and R₁₂ are hydrogen.

Another preferred aspect of the present invention is the process for preparing a compound of formula (III) or (IIIc), wherein, R₁, R₂, R₈, R₁₁, and R₁₂ are hydrogen; R₃ is hydrogen, fluoro, chloro, bromo, cyano, methyl, ethyl, ethenyl, ethynyl, or trifluoromethyl; R₄ and R₅ are independently hydrogen, fluoro, chloro, cyano, methyl, ethyl, or trifluoromethyl; R₆ is hydrogen, fluoro, chloro, bromo, methyl, methoxy, diethylamino, pyrrolidinyl, piperidyl, or morpholinyl; R₇ is isopropyl, t-butyl, or trifluoromethyl; R₉ is hydrogen or trifluoromethyl; and R₁₀ is methyl.

Hereinafter, the formulating methods and kinds of excipients will be described, but the present invention is not limited to them.

A compound of formula (I), (II), (III), (IV) or (V), an isomer thereof or a pharmaceutically acceptable salt thereof according to the present invention can be prepared as a pharmaceutical composition containing pharmaceutically acceptable carriers, adjuvants, diluents and the like. For instance, the compounds of the present invention can be dissolved in oils, propylene glycol or other solvents which are commonly used to produce an injection. Suitable examples of the carriers include, but not limited to, physiological saline, polyethylene glycol, ethanol, vegetable oils, isopropyl myristate, etc. For topical administration, the compounds of the present invention can be formulated in the form of ointment or cream.

The compound according to the present invention may also be used in the forms of pharmaceutically acceptable salts thereof, and may be used either alone or in combination or in admixture with other pharmaceutically active compounds.

The compounds of the present invention may be formulated into injections by dissolving, suspending or emulsifying in water-soluble solvent such as saline and 5% dextrose, or in water-insoluble solvents such as vegetable oils, synthetic fatty acid glyceride, higher fatty acid esters and propylene glycol. The formulations of the invention may include any of conventional additives such as dissolving agents, isotonic agents, suspending agents, emulsifiers, stabilizers and preservatives.

The preferable dose level of the compounds according to the present invention depends upon a variety of factors including the condition and body weight of the patient, severity of the particular disease, dosage form, and route and period of administration, but may appropriately be chosen by those skilled in the art. The compounds of the present invention are preferably administered in an amount ranging from 0.001 to 100 mg/kg of body weight per day, and more preferably from 0.01 to 30 mg/kg of body weight per day. Doses may be administered once a day, or several times a day with each divided portions. The compounds of the present invention are used in a pharmaceutical composition in an amount of 0.0001~10% by weight, and preferably 0.001~1% by weight, based on the total amount of the composition.

The pharmaceutical composition of the present invention can be administered to a mammalian subject such as rat, mouse, domestic animals, human being and the like via various routes. The methods of administration which may easily be expected include oral and rectal administration; intravenous, intramuscular, subcutaneous, intrauterine, duramatral and intracerebroventricular injections.

### Detailed description of the invention definitions

When describing the compounds, pharmaceutical compositions containing such compounds, methods of using such compounds and compositions, and use of such compounds and compositions, all terms used in the present application shall have the meaning usually employed by a relevant person skilled in the art, e.g. by a medicinal chemists, pharmacist or physician. By the way of example some definitions of specific groups are given below:
"Alkyl" includes monovalent saturated aliphatic hydrocarbyl groups. The hydrocarbon chain may be either straight-chained or branched. This term is exemplified by groups such as methyl, ethyl, n-propyl, isopropyl, n-butyl, iso-butyl, tert-butyl, t-amyl, and the like.
"Alkoxy" includes the group -OR wherein R is alkyl. Particular alkoxy groups include, by way of example, methoxy, ethoxy, n-propoxy, isopropoxy, n-butoxy, tert-butoxy, sec-butoxy, n-pentoxy, 1,2-dimethylbutoxy, and the like.
"Alkoxyalkoxy" refers to the group -OROR', wherein R and R' are the same or different "alkyl" groups as defined further above.
"Alkoxyalkoxyalkyl" refers to the group -ROR'OR", wherein R, R' and R" are the same or different "alkyl" groups as defined further above.
"Alkoxyalkylamino" refers to the group 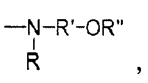 wherein R may be hydrogen or "alkyl", and R' and R" are both "alkyl" as defined further above.
"Alkoxycarbonyl" refer to the radical -C(=O)-O-R, wherein R is an alkyl group as defined herein.
"Alkenyl" includes monovalent olefinically unsaturated hydrocarbyl groups being straight-chained or branched and having at least 1 double bond. Particular alkenyl groups include ethenyl (-CH=CH₂), n-propenyl (-CH₂CH=CH₂), isopropenyl (C (CH₃) =CH₂), and the like. A preferred "alkenyl" group is ethenyl (vinyl).
"Alkynyl" includes acetylenically unsaturated hydrocarbyl groups being straight-chained or branched and having at least 1 triple bond .A preferred alkynyl group is ethynyl (acetylenyl).
"Alkylamino" includes the group -NHR', wherein R' is alkyl group as defined herein.
"Dialkylamino" includes the group -NR'R", wherein R' and R" are alkyl group as defined herein.
"Alkylsulfonyl" includes a radical-S(O)₂R, wherein R is an alkyl group as defined herein. Representative examples include, but are not limited to, methanesulfonyl, ethylsulfonyl, propylsulfonyl, butylsulfonyl and the like.
"Alkylthio" includes a radical-S-R wherein R is an alkyl group as defined herein that may be optionally substituted as defined herein. Representative examples include, but are not limited to, methylthio, ethylthio, propylthio, butylthio, and the like.
"Amino" refers to the radical-NH_{2.}
"Carboxy" refers to the radical -C(=O)OH.
"Cycloalkyl" refers to cyclic saturated aliphatic hydrocarbyl groups. The numbers of C-atoms referenced in connection with a given cycloalkyl group corresponds to the number of ring forming carbon atoms, e.g. "C3-C6 cycloalkyl" refers to a cycloalkyl with between three and six ring-forming C atoms. Examples of "cycloalkyl" are cyclopropyl, cyclobutyl, cyclopentyl, cyclohexyl etc. If indicated, a "cycloalkyl" group may be unsubstituted or substituted with one or more alkyl groups, e.g. with C1-C6 alkyl groups, preferably with C1-C3 alkyl groups, particularly preferably with methyl groups. If a "cycloalkyl" carries more than one alkyl substituent these substituents may be attached to the same or to different ring-forming carbon atoms.
"Cyano" refers to the radical -C≡N.
"Ethenyl" refers to -CH=CH₂ which is also designated "vinyl" in the present application.
"Ethynyl" refers to -C≡CH.
"Halo" or "halogen" refers to fluoro, chloro, bromo and iodo. Preferred halo groups are either fluoro or chloro.
"Haloalkyl" includes an "alkyl" group as defined further above which is substituted with one or more halogens which may be the same, e.g. in trifluoromethyl or pentafluoroethyl, or which may be different.
"Hydroxy" refers to the radical-OH.
"Hydroxyalkyl" includes an "alkyl" group as defined further above which is substituted with one or more hydroxy groups.
"Nitro" refers to the radical-NO₂.
"Alkylpiperazinyl" refers to a piperazine ring that carries an "alkyl" as substituent, wherein the piperazinyl ring is preferably bound both to the "alkyl" as well as to the second attachment position via its nitrogen atoms.

"Pharmaceutically acceptable" means being approved by a regulatory agency of the Federal or a state government or being listed in the U. S. Pharmacopoeia or other generally recognized pharmacopoeia for use in animals, and more particularly in humans.

"Pharmaceutically acceptable salt" refers to a salt of a compound of the invention that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid,3- (4-hydroxybenzoyl) benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2naphthalenesulfonic acid, 4-toluenesulfonic acid,camphorsulfonic acid, 4methylbicyclo [2.2.2]-oct-2-ene-1-carboxylic acid, glucoheptonic acid,3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound is replaced.

"Pharmaceutically acceptable carrier" refers to a diluent, adjuvant, excipient or carrier with which a compound of the invention is administered.

"Preventing" or "prevention" refers to a reduction in risk of acquiring a disease or disorder (i. e., causing at least one of the clinical symptoms of the disease not to develop in a subject that may be exposed to or predisposed to the disease but does not yet experience or display symptoms of the disease).

"Subject" includes humans. The terms "human," "patient" and "subject" are used interchangeably herein.

"Therapeutically effective amount" means the amount of a compound that, when administered to a subject for treating a disease, is sufficient to effect such treatment for the disease. The"therapeutically effective amount" can vary depending on the compound, the disease and its severity, and the age, weight, etc., of the subject to be treated.

"Treating" or "treatment" of any disease or disorder refers, in one embodiment, to ameliorating the disease or disorder (i. e., arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically, (e. g., stabilization of a discernible symptom), physiologically, (e. g., stabilization of a physical parameter), or both. In yet another embodiment, "treating" or "treatment" refers to delaying the onset of the disease or disorder.

### Mode for carrying out invention

The present invention is more specifically explained by following examples and experimental examples. However, it should be understood that the extent of the present invention is not limited to the following examples and experimental examples

### Example 1: 3-(6-tert-Butyl-pyridin-3-yl)-N-(3-fluoro-4-methanesulfonylamino-benzyl)-acrylamide

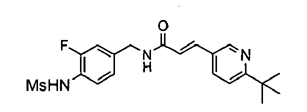

### Step 1: Synthesis of 3-(6-tert-Butyl-pyridin-3-yl)-acrylic acid

To a solution of 6-tert-Butyl-pyridine-3-carboxaldehyde (1.34g, 8.75mmol) prepared by known procedure in toluene was added methyl (triphenylphosphoranylidene)acetate (2.93g), and the resulting was heated at 90°C for 3 hrs. The reaction mixture was diluted with EtOAc, and washed with water and brine. The organic layer was dried over anhydrous MgSO₄ and concentrated under reduced pressure. The resulting residue was purified by column chromatography (Hex/EtOAc = 4/1) to give ester product (1.56g, 81%). The resulting ester was dissolved in 1,4-dioxane, treated with water and KOH, stirred and heated at reflux for 18hrs. The reaction mixture was cooled to room temperature, diluted with water, and then washed with ether. The aqueous phase was acidified with 1N HCl, and then extracted with CHCl₃, and the combined organic phase was washed with brine, dried over anhydrous MgSO₄ and concentrated under reduced pressure to give 3-(6-tert-Butyl-pyridin-3-yl)-acrylic acid (1.00g, 68%).
¹H NMR(300MHz, CDCl₃): δ 8.78(d, 1H, *J*=2.1Hz), 7.84(dd, 1H, *J*=2.1 and 8.4Hz), 7.78(d, 1H, *J*=16.2Hz), 7.42(d, 1H, *J*=8.4Hz), 6.53(d, 1H, *J*=16.2Hz), 1.40(s, 9H)

### Step 2: Synthesis of 3-(6-tert-Butyl-pyridin-3-yl)-N-(3-fluoro-4-methanesulfonylaminobenzyl)-acrylamide

N-(4-Aminomethyl-2-fluoro-phenyl)-methanesulfonamide, HCl salt (50mg, 0.20mmol) was reacted with 3-(6-tert-Butyl-pyridin-3-yl)-acrylic acid (40mg) to give 3-(6-tert-Butyl-pyridin-3-yl)-N-(3-fluoro-4-methanesulfonylamino-benzyl)-acrylamide (75mg, 92%) after purification by column chromatography (Hex/EtOAc = 1/2).
¹H NMR(300MHz, CDCl₃): δ 8.70(d, 1H, *J=*2.1Hz), 7.77(dd, 1H, *J=*2.1 and 8.1Hz), 7.64(d, 1H, *J*=15.6Hz), 7.48(m, 1H), 7.38(d, 1H, *J*=8.4Hz), 7.13(m, 2H), 6.77(s, 1H), 6.51(d, 1H, *J*=15.6Hz), 6.43(t, 1H), 4.54 (d, 2H, *J*=6.0Hz), 3.02(s, 3H), 1.38(s, 9H) ESI [M+H]⁺: 406.2.

### Example 2: 3-(6-tert-Butyl-pyridin-3-yl)-N-(3-chloro-4-methanesulfonylamino-benzyl)-acrylamide

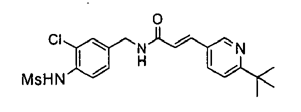

N-(4-Aminomethyl-2-chloro-phenyl)-methanesulfonamide, HCl salt (100mg, 0.35mmol) was reacted with 3-(6-tert-Butyl-pyridin-3-yl)-acrylic acid (70mg) to give 3-(6-tert-Butyl-pyridin-3-yl)-N-(3-chloro-4-methanesulfonylamino-benzyl)-acrylamide (110mg, 74%) after purification by column chromatography (Hex/EtOAc = 1/2).
¹H NMR(300MHz, CDCl₃): δ 8.66(d, 1H, *J*=2.1Hz), 7.74(dd, 1H, *J*=2.1 and 8.1Hz), 7.64(d, 1H, *J*=15.6Hz), 7.57(d, 1H, *J* =8.7Hz), 7.41(d, 1H, *J*=2.1Hz), 7.36(d, 1H, *J* =8.1Hz), 7.24(dd, 1H, *J*=2.1 and 8.7Hz), 6.82(s, 1H), 6.48(d, 1H, *J*=15.6Hz), 6.42(t, 1H), 4.53 (d, 2H, *J*=6.0Hz), 3.00(s, 3H), 1.37(s, 9H)
ESI [M+H]⁺: 422.2.

### Example 3: 3-(6-tert-Butyl-pyridin-3-yl)-N-(3-ethynyl-5-fluoro-4-methanesulfonylamino-benzyl)-acrylamide

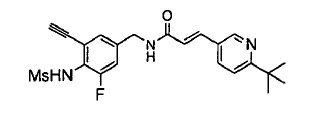

N-(4-Aminomethyl-2-ethynyl-6-fluoro-phenyl)-methanesulfonamide, HCl salt (70mg, 0.25mmol) was reacted with 3-(6-tert-Butyl-pyridin-3-yl)-acrylic acid (52mg) to give 3-(6-tert-Butyl-pyridin-3-yl)-N-(3-ethynyl-5-fluoro-4-methanesulfonylamino-benzyl)-acrylamide (63mg, 64%) after purification by column chromatography (Hex/EtOAc = 1/2).
¹H NMR(300MHz CDC1₃): δ 8.71(d, 1H, *J* =2.4Hz), 7.76(dd, 1H, *J* =2.4 and 8.4Hz), 7.63(d, 1H, *J* =16.0Hz), 7.39(d, 1H, *J* =8.4Hz), 7.28(s, 1H), 7.16(dd, 1H, *J =2.1* and 11.0Hz), 6.64(s, 1H), 6.52(d, 1H, *J* =16.0Hz), 6.47(t, 1H), 4.51 (d, 2H, *J* =6.0Hz), 3.45(s, 1H), 3.24(s, 3H), 1.38(s, 9H)
ESI [M+H]⁺: 430.1.

### Example 4: 3-(6-tert-Butyl-pyridin-3-yl)-N-(4-methanesulfonylamino-3-vinyl-benzyl)-acrylamide

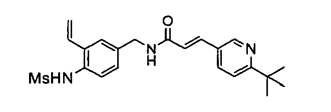

N-(4-Aminomethyl-2-vinyl-phenyl)-methanesulfonamide, HCl salt (70mg, 0.28mmol) was reacted with 3-(6-tert-Butyl-pyridin-3-yl)-acrylic acid (52mg) to give 3-(6-tert-Butyl-pyridin-3-yl)-N-(4-methanesulfonylamino-3-vinyl-benzyl)-acrylamide (62mg, 54%) after purification by column chromatography (Hex/EtOAc = 1/2).
¹H NMR(300MHz, CDCl₃): δ 8.66(d, 1H, *J* =1.8Hz), 7.74(dd, 1H, *J =2.1* and 8.4Hz), 7.63(d, 1H, *J* =16.0Hz), 7.44(d, 1H, *J* =2.1Hz), 7.36(m, 2H), 7.23(m, 2H), 6.90(dd, 1H, *J* =11.0 and 17.0Hz), 6.70(s, 1H), 6.48(d, 1H, *J* =16.0Hz), 6.40(t, 1H), 5.70(d, 1H, *J* =17.0Hz), 5.43(d, 1H, *J =*11.0Hz), 4.54(d, 2H, *J* =5.7Hz), 2.98(s, 3H), 1.37(s, 9H)
ESI [M+H]⁺: 414.2.

### Example 5: N-(3-Fluoro-4-methanesulfonylamino-benzyl)-3-(2-morpholin-4-yl-6-trifluoromethyl-pyridin-3-yl)-acrylamide

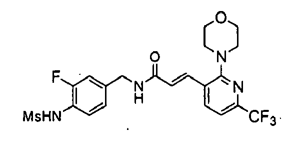

N-(4-Aminomethyl-2-fluoro-phenyl)-methanesulfonamide, HCl salt(100mg, 0.40mmol) was reacted with 3-(2-Morpholin-4-yl-6-trifluoromethyl-pyridin-3-yl)-acrylic acid (121mg) prepared by known procedure to give N-(3-Fluoro-4-methanesulfonylamino-benzyl)-3-(2-morpholin-4-yl-6-trifluoromethyl-pyridin-3-yl)-acrylamide (118mg, 66%) after purification by column chromatography (CH₂Cl₂/MeOH = 20/1).
¹H NMR(300MHz, CDCl₃ + DMSO-d₆): δ 9.13(s, 1H), 8.32(t, 1H), 7.96(d, 1H, *J* =7.8Hz), 7.79(d, 1H, *J* =1 5.9Hz), 7.56(t, 1H, *J* =8.4Hz), 7.38(d, 1H, *J*=7.8Hz), 7.26(m, 2H), 6.80(d, 1H, *J* =15.9Hz), 4.63(d, 2H, *J* =5.7Hz), 3.98(m, 4H), 3.46(m, 4H), 3.13(s, 3H)
ESI [M+H]⁺: 503.1.

### Example 6: N-(4-Methanesulfonylamino-3-vinyl-benzyl)-3-(2-morpholin-4-yl-6-trifluoromethyl-pyridin-3-yl)-acrylamide

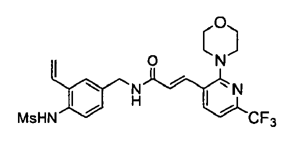

N-(4-Aminomethyl-2-vinyl-phenyl)-methanesulfonamide, HCl salt (70mg, 0.27mmol) was reacted with 3-(2-Morpholin-4-yl-6-trifluoromethyl-pyridin-3-yl)-acrylic acid (81mg) to give N-(4-Methanesulfonylamino-3-vinyl-benzyl)-3-(2-morpholin-4-yl-6-trifluoromethyl-pyridin-3-yl)-acrylamide (55mg, 45%) after purification by column chromatography (CH₂Ch/MeOH = 20/1).
¹H NMR(300MHz, CDCl₃ + DMSO-d₆): δ 8.92(s, 1H), 8.18(s, 1H), 7.97(d, 1H, *J* =7.8Hz), 7.83(d, 1H, *J* =16.0Hz), 7.73(s, 1H), 7.44(m, 3H), 7.33(dd, 1H, *J* =11.0 and 17.0Hz), 6.83(d, 1H, *J* =16.0Hz), 5.93(d, 1H, *J* =17.0Hz), 6.55(d, 1H, *J* =11.0Hz), 4.69(d, 2H, *J* =5.1Hz), 4.01 (m, 4H), 3.50(m, 4H), 3.09(s, 3H)
ESI [M+H]⁺: 511.1.

### Example 7: N-(3-Chloro-4-methanesulfonylamino-benzyl)-3-(2-morpholin-4-yl-6-trifluoromethyl-pyridin-3-yl)-acrylamide

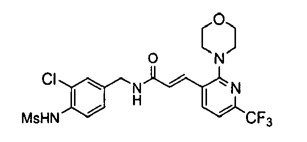

N-(4-Aminomethyl-2-chloro-phenyl)-methanesulfonamide, HCl salt (62mg, 0.22mmol) was reacted with 3-(2-Morpholin-4-yl-6-trifluoromethyl-pyridin-3-yl)-acrylic acid (67mg) to give N-(3-Chloro-4-methanesulfonylamino-benzyl)-3-(2-morpholin-4-yl-6-trifluoromethyl-pyridin-3-yl)-acrylamide (93mg, 91%) after purification by column chromatography (CH₂Cl₂/MeOH = 20/1).
¹H NMR(300MHz, CDCl₃ + DMSO-d₆): δ 8.37(s, 1H), 8.27(t, 1H), 7.99(d, 1H, *J* =7.8Hz), 7.86(d, 1H, *J* =15.9Hz), 7.72(d, 1H, *J* =8.4Hz), 7.62(m, 1H), 7.44(m, 2H), 6.84(d, 1H, *J*=15.9Hz), 4.68(d, 2H, *J*=5.7Hz), 4.04(m, 4H), 3.52(m, 4H), 3.19(s, 3H)
ESI [M+H]⁺: 519.1.

### Example 8: N-(3-Fluoro-4-methanesulfonylamino-5-methyl-benzyl)-3-(2-morpholin-4-yl-6-trifluoromethyl-pyridin-3-yl)-acrylamide

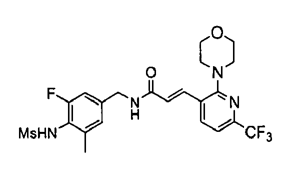

N-(4-Aminomethyl-2-fluoro-6-methyl-phenyl)-methanesulfonamide, HCl salt (100mg, 0.35mmol) was reacted with 3-(2-Morpholin-4-yl-6-trifluoromethyl-pyridin-3-yl)-acrylic acid (116mg) to give N-(3-Fluoro-4-methanesulfonylamino-5-methyl-benzyl)-3-(2-morpholin-4-yl-6-trifluoromethyl-pyridin-3-yl)-acrylamide (120mg, 74%) after purification by column chromatography (Hex/EtOAc = 1/2).
¹H NMR(300MHz, CDCl₃): δ 7.76(d, 1H, *J* =7.8Hz), 7.71(d, 1H, *J* =15.6Hz), 7.24(m, 3H), 6.42(d, 1H, *J* =15.6Hz), 6.32(s, 1H), 6.06(t, 1H), 4.59(d, 2H, *J* =6.3Hz), 3.85(m, 4H), 3.34(m, 4H), 3.05(s, 3H), 2.25(d, 3H, *J* =2.1Hz)
ESI [M+H]⁺: 517.1.

### Example 9: 3-(6-tert-Butyl-2-methoxy-pyridin-3-yl)-N-(3-fluoro-4-methanesulfonylamino-benzyl)-acrylamide

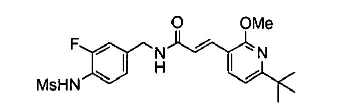

3-fluoro-4-methanesulfonylaminobenzylamine hydrochloride(17.8mg, 0.052mmol) was reacted with 3-(6-tert-Butyl-2-methoxy-pyridin-3-yl)-acrylic acid (12mg) DMTMM(1.1eq, 16mg) and NMP(1.2eq, 90µl) in THF to give title compound (14mg, 61.8%) after purification by column chromatography (Hex/EtOAc = 3/2).
¹H NMR(300MHz, CDCl₃): δ 7.70(d, 1H, *J* =15.6Hz) 7.61(d, 1H, *J* =9.3Hz) 7.51(m, 1H) 7.13(m, 2H) 6.90(d, 1H, *J* =7.8Hz) 6.84(d, 1H, *J* =15.6Hz) 6.61(bs, 1H) 6.10 (bs, 1H) 4.54(d, 2H, *J*=6Hz) 4.01(s, 3H) 3.02(s, 3H) 1.33(s, 9H)
ESI [M+H]⁺: 436.1.

### Example 10: 3-(6-tert-Butyl-pyridin-3-yl)-N-(3-fluoro-4-methanesulfonylamino-5-vinyl-benzyl)-acrylamide

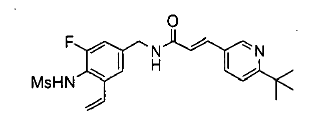

N-(4-Aminomethyl-2-fluoro-6-vinyl-phenyl)-methanesulfonamide, HCl salt (84mg, 0.30mmol) was reacted with 3-(6-tert-Butyl-pyridin-3-yl)-acrylic acid (62mg) to give 3-(6-tert-Butyl-pyridin-3-yl)-N-(3-fluoro-4-methanesulfonylamino-5-vinyl-benzyl)-acrylamide (34mg, 26%) after purification by column chromatography (Hex/EtOAc = 1/2).
¹H NMR(300MHz, CDCl₃+ DMSO-d₆): δ 8.68(d, 1H, *J* =2.4Hz), 8.09(s, 1H), 7.78(dd, 1H, *J* =2.1 and 8.4Hz), 7.61(s, 1H), 7.56(d, 1H, *J* =15.9Hz), 7.35(d, 1H, *J* =8.4Hz), 7.33(s, 1H), 7.12(dd, 1H, *J*=14.8 and 18.0Hz), 7.01(dd, 1H, *J*=1.8 and 10.2Hz), 6.60(d, 1H, *J* =15.9Hz), 5.73(d,1H, *J* =18.0Hz), 5.33(d, 1H, *J* =11.4Hz), 4.46(d, 2H, *J* =6.0Hz), 2.97(d, 3H, *J*=0.9Hz), 1.34(s, 9H)
ESI [M+H]⁺: 432.2.

### Example 11: 3-(6-tert-Butyl-2-methoxy-pyridin-3-yl)-N-(3-ethynyl-5-fluoro-4-methanesulfonylamino-benzyl)-acrylamide

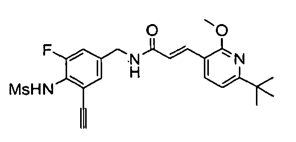

N-(4-Aminomethyl-2-ethynyl-6-fluoro-phenyl)-methanesulfonamide, HCl salt(82.4mg, 0.30mmol) was reacted with 3-(6-tert-Butyl-2-methoxy-pyridin-3-yl)-acrylic acid (66mg), DMTMM(1.1eq, 90mg) and NMP(1.2eq, 40µl) in THF to give the title compound (51.2mg, 37.6%) after purification by column chromatography (Hex/EtOAc = 1/1).
¹H NMR(300MHz, CDCl₃): δ 7.70(d, 1H, *J*=15.9Hz) 7.62(d, 1H, *J*=8.4Hz) 7.30(s, 1H) 7.17(m, 1H) 6.91(d, 1H, *J*=7.8Hz) 6.69(d, 1H, *J* =15.6Hz) 6.41(s, 1H) 6.02 (bs, 1H) 4.53(d, 2H, *J* =6Hz) 4.02(s, 3H) 3.47(s, 1H) 3.26(s, 3H) 1.34(s, 9H)
ESI [M+H]⁺: 460.1.

### Example 12: N-(3-Ethynyl-5-fluoro-4-methanesulfonylamino-benzyl)-3-(2-morpholin-4-yl-6-trifluoromethyl-pyridin-3-yl)-acrylamide

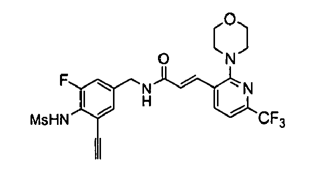

N-(4-Aminomethyl-2-ethynyl-6-fluoro-phenyl)-methanesulfonamide, HCl salt (100mg, 0.22mmol) was reacted with 3-(2-Morpholin-4-yl-6-trifluoromethyl-pyridin-3-yl)-acrylic acid (108mg) to give N-(3-Ethynyl-5-fluoro-4-methanesulfonylamino-benzyl)-3-(2-morpholin-4-yl-6-trifluoromethyl-pyridin-3-yl)-acrylamide (190mg, 100%) after purification by column chromatography (Hex/EtOAc = 1/2).
¹H NMR(300MHz, CDCl₃ + DMSO-d₆): δ 9.31(s, 1H), 8.73(t, 1H), 7.92(d, 1H, *J* =7.8Hz), 7.49(d, 1H, *J* =15.6Hz), 7.31(d, 1H, *J* =7.8Hz), 7.25(s, 1H), 7.18(d, 1H, *J* =10.8Hz), 6.71(d, 1H, *J* =15.6Hz), 4.38(d, 2H, *J* =5.7Hz), 4.14(s, 1H), 3.76(m, 4H), 3.22(m, 4H), 3.04(s, 3H)
ESI [M+H]⁺: 527.2.

### Example 13: 2-(6'-Chloro-3,4,5,6-tetrahydro-2H-[1,2']bipyridinyl-3'-yloxy)-N-(3-fluoro-4-methanesulfonylamino-benzyl)-acetamide

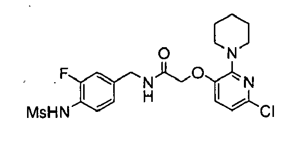

N-(4-Aminomethyl-3-fluoro-phenyl)-methanesulfonamide HCl salt (32 mg, 0.12 mmol) and NMP (0.05 ml) were added in 20 ml of THF. The mixture was stirred for 10 mins. DMTMM (51 mg, 0.18 mmol) and 3-(6'-Chloro-3,4,5,6-tetrahydro-2H-[1,2']bipyridinyl-3'-yl)-acrylic acid (20 mg, 0.092 mmol) were added into the mixture. The reaction mixture was stirred overnight. The reaction solvent was removed in vacuo. The residue was extracted with ethylacetate (30 ml x 3) and H₂O (30 ml). A combined organic layer was washed with sat. NaHCO₃ (30ml), and with brine (30 ml), dried with MgSO₄, and concentrated in vacuo. The residue was purified with column chromatography to yield a white solid (11 mg).
¹H NMR (CDCl₃, 300 MHz) δ 7.87 (d, *J* = 15.9 Hz, 1H), 7.62 (d, *J* = 9.0 Hz, 1H), 7.51 (t, j = 7.8 Hz, 1H), 7.12 (m, 2H), 6.39 (br, 1H), 6.51 (d, *J* = 9.0 Hz, 1H), 6.20 (d, *J* = 15.6 Hz), 6.00 (br, 1H), 5.53 (d, *J* = 6.3 Hz, 2H), 3.60 (m, 4H), 3.01 (s, 3H), 1.65 (m, 6H)
ESI [M+H]⁺: 467.1.

### Example 14: 3-(6-Chloro-pyridin-3-yl)-N-(3-ethynyl-5-fluoro-4-methanesulfonylamino-benzyl)-acrylamide

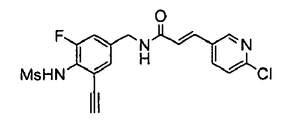

### Step 1: 3-(6-Chloro-pyridin-3-yl)-acrylic acid methyl ester

To 6-Chloro-pyridine-3-carboxaldehyde (300 mg, 2.12 mmol) in toluene was added Methyl(Triphenylphosphoranylidene)acetate (708 mg, 2.12 mmol) and the solution was refluxed for 6 hrs. The reaction mixture was diluted with EtOAc and then washed three times with H₂O, brine, dried. Na₂SO₄, filtered and concentrated under reduced pressure. The obtained residue was column-chromatographed to yield 3-(6-Chloro-pyridin-3-yl)-acrylic acid methyl ester (380 mg, 90 %).
¹H NMR (300 MHz, CDCl₃): δ 8.51 (d, 1H, *J =* 2.7 Hz), 7.80 (dd, 1H, *J=* 8.1, 2.4 Hz), 7.65 (d, 1H, *J* = 15.9 Hz), 7.36 (d, 1H, *J* = 8.4 Hz), 6.48 (d, 1H, *J* = 15.9 Hz), 3.83 (s, 3H).

### Step 2: 3-(6-Chloro-pyridin-3-yl)-acrylic acid

3-(6-Chloro-pyridin-3-yl)-acrylic acid methyl ester (107 mg, 0.541 mmol) in THF was added to a solution of 0.5 N-LiOH (2 eq) and the mixture was stirred for 3 hrs at room temperature. The resulting residue was dissolved in H₂O and then washed three times with Et₂O, neutralized with 1N HCl to pH 5~7. The resulting solid filtered and washed with H₂O and then dried in vacuo to give 3-(6-Chloro-pyridin-3-yl)-acrylic acid (80 mg, 80 %).
¹H NMR (300 MHz, DMSO-d⁶): δ 8.64 (s, 1H), 8.16 (d, 1H, *J* = 8.1 Hz), 7.54 (d, 1H, *J=*16.8 Hz), 7.50 (d, 1H, *J* = 9.9 Hz), 6.63 (d, 1H, *J* = 15.9 Hz).

### Step 3: 3-(6-Chloro-pyridin-3-yl)-N-(3-ethynyl-5-fluoro-4-methanesulfonylamino-benzyl)-acrylamide

N-(4-Aminomethyl-2-ethynyl-6-fluoro-phenyl)-methanesulfonamide HCl salt (78.3 mg, 0.272 mmol) was suspended in THF and treated with triethylamine (30 mg, 0.299 mmol) and then the resulting mixture was stirred for 10mins. 3-(6-Chloro-pyridin-3-yl)-acrylic acid (50 mg, 0.272 mmol) was added to the reaction mixture followed by DMTMM (82 mg, 0.299 mmol) after 10 mins. The resulting mixture was stirred overnight at ambient temperature and then diluted with EtOAc. The resulting solution was washed successively with water, sat'd NaHCO₃ (x2), brine, and then dried over anhyd. Na₂SO₄, filtered and concentrated under reduced pressure. The crude residue was recrystallized (CH₂Cl₂) to yield the title compound (26 mg, 23 %).
¹H NMR (300 MHz, DMSO-d⁶): δ 9.54 (s, 1H), 8.74 (t, 1H, *J*= 6.0 Hz), 8.61 (d, 1H, *J =* 2.4 Hz), 8.07 (dd, 1H, *J =* 8.4, 2.4 Hz), 7.56 (d, 1H, *J* = 8.4 Hz), 7.51 (d, 1H, *J* = 15.9 Hz), 7.34 (t, 1H, *J* = 8.1 Hz), 7.19 (d, 1H, *J* = 12.0 Hz), 7.12 (d, 1H, *J* = 8.4 Hz), 6.79 (d, 1H, *J* = 15.9 Hz), 4.39 (d, 2H, *J* = 5.7 Hz), 3.00 (s, 3H).
ESI [M+H]⁺; 408.0.

### Example 15: 3-(6-Bromo-pyridin-3-yl)-N-(3-ethynyl-5-fluoro-4-methanesulfonylamino-benzyl)-acrylamide

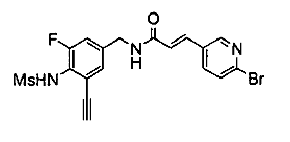

### Step 1: 3-(6-Bromo-pyridin-3-yl)-acrylic acid methyl ester

To 6-Bromo-pyridine-3-carboxaldehyde (300 mg, 1.61 mmol) in toluene was added Methyl(Triphenylphosphoranylidene)acetate (647 mg, 1.94 mmol) and the solution was refluxed for 6 hrs. The reaction mixture was diluted with EtOAc and then washed three times with H₂O, brine, dried. Na₂SO₄, filtered and concentrated under reduced pressure. The obtained residue was column-chromatographed to yield 3-(6-Bromo-pyridin-3-yl)-acrylic acid methyl ester (380 mg, 97 %).
¹H NMR (300 MHz, CDCl₃): δ 8.47 (d, 1H, *J=*2.4 Hz), 7.68 (dd, 1H, *J=*8.4, 2.4 Hz), 7.60 (d, 1H, *J=*15.9 Hz), 7.51 (d, 1H, *J=*8.4 Hz), 6.49 (d, 1H, *J*=15.9 Hz), 3.81 (s, 3H).

### Step 2: 3-(6-Bromo-pyridin-3-yl)-acrylic acid

To 3-(6-Bromo-pyridin-3-yl)-acrylic acid methyl ester (120 mg, 0.495 mmol) in THF was added a solution of 0.5 N-LiOH (2 eq) and the mixture was stirred for 3 hrs at room temperature. The resulting residue was dissolved in H₂O, then washed three times with Et₂O, and neutralized with 1N HCl to pH 5~7. The resulting solid filtered and washed with H₂O and then dried in vacuo to give 3-(6-Bromo-pyridin-3-yl)-acrylic acid (100 mg, 88 %).
¹H NMR (300 MHz, DMSO-d⁶): δ 8.67 (d, 1H, *J*=2.1 Hz), 8.11 (dd, 1H, *J*=8.4, 2.1 Hz), 7.69 (d, 1H, *J*=8.4 Hz), 7.59 (d, 1H, *J*=15.9 Hz), 6.71 (d, 1H, *J*=15.9 Hz).

### Step 3: 3-(6-Bromo-pyridin-3-yl)-N-(3-ethynyl-5-fluoro-4-methanesulfonylamino-benzyl)-acrylamide

N-(4-Aminomethyl-2-ethynyl-6-fluoro-phenyl)-methanesulfonamide HCl salt (63 mg, 0.219 mmol) was suspended in THF and treated with triethylamine (25 mg, 0.241 mmol) and then the resulting mixture was stirred for 10mins. 3-(6-Bromo-pyridin-3-yl)-acrylic acid (50 mg, 0.219 mmol) was added to the reaction mixture followed by DMTMM (66 mg, 0.241 mmol) after 10 mins. The resulting mixture was stirred overnight at ambient temperature and then diluted with EtOAc. The resulting solution was washed successively with water, sat'd NaHCO₃ (x2), and brine, and then dried over anhyd. Na₂SO₄, filtered and concentrated under reduced pressure. The crude residue was recrystallized (EtOAc/n-Hexane) to yield the title compound (71 mg, 72 %).
¹H NMR (300 MHz, DMSO-d⁶): δ 9.42 (s, 1H), 8.77 (t, 1H, *J* = 6.0 Hz), 8.60 (d, 1H, *J* = 2.4 Hz), 7.96 (dd, 1H, *J* = 8.1, 1.8 Hz), 7.70 (d, 1H, *J* = 8.1 Hz), 7.48 (d, 1H, *J* = 15.9 Hz), 7.28 (s, 1H), 7.27 (d, 1H, *J =* 8.7 Hz), 6.81 (d, 1H, *J =* 15.9 Hz), 4.50 (s, 1H), 4.39 (d, 2H, *J* = 5.7 Hz), 3.06 (s, 3H).
ESI [M+H]⁺; 452.0.

### Example 16: 3-(6-Chloro-pyridin-3-yl)-N-(3-fluoro-4-methanesulfonylamino-benzyl)-acrylamide

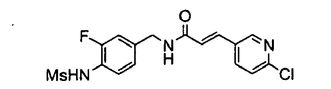

3-fluoro-4-methanesulfonylaminobenzylamine hydrochloride (51 mg, 0.201 mmol) was suspended in THF and treated with triethylamine (23 mg, 0.22 mmol) and then the resulting mixture was stirred for 10mins. 3-(6-Chloro-pyridin-3-yl)-acrylic acid (37 mg, 0.201 mmol) was added to the reaction mixture followed by DMTMM (61 mg, 0.22 mmol) after 10 mins. The resulting mixture was stirred overnight at ambient temperature and then diluted with EtOAc. The resulting solution was washed successively with water, sat'd NaHCO₃ (x2), brine, and then dried over anhyd. Na₂SO₄, filtered and concentrated under reduced pressure. The crude residue was column-chromatographed to yield the title compound (74 mg, 96 %).
¹H NMR (300 MHz, DMSO-d⁶): δ 9.54 (s, 1 H), 8.74 (t, 1H, *J =* 6.0 Hz), 8.62 (d, 1H, *J* = 2.4 Hz), 8.07 (dd, 1H, *J* = 6.0, 2.4 Hz), 7.57 (d, 1H, *J* = 8.4 Hz), 7.51 (d, 1H, *J* = 15.9 Hz), 7.34 (t, 1H, *J* = 8.1 Hz), 7.19 (d, 1H, *J* = 12.0 Hz), 7.12 (d, 1H, *J* = 8.4 Hz), 6.79 (d, 1H, *J=* 15.9 Hz), 4.39 (d, 2H, *J=* 5.7 Hz), 3.00 (s, 3H).
ESI [M+H]⁺; 384.0.

### Example 17: 3-(6-tert-Butyl-4-trifluoromethyl-pyridin-3-yl)-N-(3-fluoro-4-methanesulfonylamino-benzyl)-acrylamide

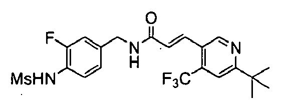

3-fluoro-4-methanesulfonylaminobenzylamine hydrochloride (41.2mg, 0.162mmol) was reacted with 3-(6-tert-Butyl-4-trifluoromethyl-pyridin-3-yl)-acrylic acid (1.0eq, 44.2mg) DMTMM(1.0eq, 44.8mg) and NMP(1.2eq, 22µl) in THF to give the title compound (48mg, 62.6%) after purification by column chromatography (Hex/EtOAc = 1/1).
¹H NMR(300MHz, CDCl₃): δ 8.84(s, 1H) 7.93(d, 1H, *J* =15.3Hz) 7.57(s, 1H) 7.52(t, 1H) 7.14(m, 1H) 6.60(bs, 1H) 6.45(d, 1H, *J* =15.6Hz) 6.24(bs, 1H) 6.50(bs, 1H) 4.54(d, 2H, *J*=6.0Hz) 3.03(s, 3H) 1.39(s, 9H)
ESI [M+H]⁺: 474.2.

### Example 18: 3-(6-tert-Butyl-4-trifluoromethyl-pyridin-3-yl)-N-(3-ethynyl-5-fluoro-4-methanesulfonylamino-benzyl)-acrylamide

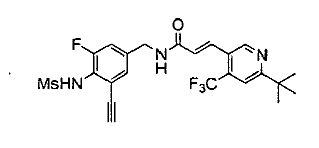

N-(4-Aminomethyl-2-ethynyl-6-fluoro-phenyl)-methanesulfonamide HCl salt (11.2mg, 0.04mmol) was reacted with 3-(6-tert-Butyl-4-trifluoromethyl-pyridin-3-yl)-acrylic acid (1.0eq, 11.0mg) DMTMM(1.0eq, 11.1mg) and NMP(1.2eq, 6µl) in THF to give the title compound (6mg, 30.2%) after purification by column chromatography (Hex/EtOAc = 3/2).
¹H NMR(300MHz, CDCl₃): δ 8.85(s, 1H) 7.95(d, 1H, *J*=13.5Hz) 7.58(s, 1H) 7.32(bs, 1H) 7.20(d, 1H, *J=*12.6Hz) 6.05(bs, 1H) 4.54(d, 1H, *J*=6.0Hz) 3.49(s, 1H) 3.27(s, 3H) 1.40(s, 9H)
ESI [M+H]⁺: 498.2

### Example 19: N-(3-Fluoro-4-methanesulfonylamino-benzyl)-3-(3,4,5,6-tetrahydro-2H-[1,2']bipyridinyl-5'-yl)-acrylamide

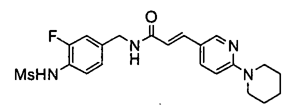

### Step 1: 3-(3,4,5,6-Tetrahydro-2H-[1,2']bipyridinyl-5'-yl)-acrylic acid methyl ester

3-(6-Bromo-pyridin-3-yl)-acrylic acid methyl ester (180 mg, 0.74 mmol) was added to piperidine (1 ml) and the mixture was stirred for 1.5 hrs at room temperature. The reaction mixture was diluted with EtOAc and then washed three times with H₂O, brine, dried. Na₂SO₄, filtered and concentrated under reduced pressure. The obtained residue was column-chromatographed to yield 3-(3,4,5,6-Tetrahydro-2H-[1,2']bipyridinyl-5'-yl)-acrylic acid methyl ester (60 mg, 33 %).
¹H NMR (300 MHz, CDCl₃): δ 8.25 (d, 1H, *J =* 2.1 Hz), 7.63 (dd, 1H, *J =* 6.0, 2.4 Hz), 7.58 (d, 1H, *J*=15.9 Hz), 6.62 (d, 1H, *J*=9.3 Hz), 6.21 (d, 1H, *J* = 15.9 Hz), 3.78 (s, 3H), 3.64 - 3.61 (m, 4H), 1.66 - 1.60 (m, 6H).

### Step 2: N-(3-Fluoro-4-methanesulfonylamino-benzyl)-3-(3,4,5,6-tetrahydro-2H-[1,2']bipyridinyl-5'-yl)-acrylamide

3-fluoro-4-methanesulfonylaminobenzylamine hydrochloride (25.2 mg, 0.099 mmol) was suspended in THF and treated with triethylamine (11 mg, 0.108 mmol) and then the resulting mixture was stirred for 10mins. 3-(3,4,5,6-Tetrahydro-2H-[1,2']bipyridinyl-5'-yl)-acrylic acid (23 mg, 0.099 mmol) was added to the reaction mixture followed by DMTMM (30 mg, 0.108 mmol) after 10 mins. The resulting mixture was stirred overnight at ambient temperature and then diluted with EtOAc. The resulting solution was washed successively with water, sat'd NaHCO₃ (x2), brine, and then dried over anhyd. Na₂SO₄, filtered and concentrated under reduced pressure. The crude residue was recrystallized (CH₂Cl₂) to yield the title compound (15 mg, 35 %).
¹H NMR (300 MHz, CDCl₃): δ 8.25 (d, 1H, *J =* 2.7 Hz), 7.59 (dd, 1H, *J =* 6.0, 2.7 Hz), 7.52 (d, 1H, *J* = 15.9 Hz), 7.15 (t, 1H, *J* = 6.0 Hz), 6.62 (d, 1H, *J* = 9.0 Hz), 6.21 (d, 1H, *J =* 15.3 Hz), 6.01 (s, 1H, br), 4.53 (d, 2H, *J =* 6.0 Hz), 3.62 - 3.59 (m, 4H), 3.01 (s, 3H), 1.65 - 1.64 (m, 6H).
ESI [M+H]⁺: 433.2.

### Example 20: 3-(6-Bromo-pyridin-3-yl)-N-(3-fluoro-4-methanesulfonylamino-benzyl)-acrylamide

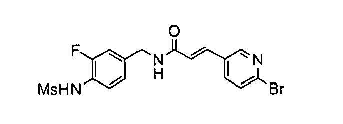

3-fluoro-4-methanesulfonylaminobenzylamine hydrochloride (183 mg, 0.719 mmol) was suspended in THF and treated with triethylamine (80 mg, 0.791 mmol) and then the resulting mixture was stirred for 10mins. (6-Chloro-pyridin-3-yloxy)-acetic acid (164 mg, 0.719 mmol) was added to the reaction mixture followed by DMTMM (218 mg, 0.791 mmol) after 10 mins. The resulting mixture was stirred overnight at ambient temperature and then diluted with EtOAc. The resulting solution was washed successively with water, sat'd NaHCO₃ (x2), brine, and then dried over anhyd. Na₂SO₄, filtered and concentrated under reduced pressure. The crude residue was recrystallized (CH₂Cl₂) to yield the title compound (225 mg, 65 %).
¹H NMR (300 MHz, DMSO-d⁶): δ 9.54 (s, 1H), 8.75 (t, 1H, *J* = 6.0 Hz), 8.59 (d, 1H, *J =* 2.1 Hz), 7.95 (dd, 1H, *J =* 8.4, 2.4 Hz), 7.71 (d, 1H, *J =* 8.4 Hz), 7.49 (d, 1H, *J =* 15.9 Hz), 7.34 (t, 1H, *J* = 8.1 Hz), 7.19 (d, 1H, *J* = 12.0 Hz), 7.12 (d, 1H, *J* = 8.4 Hz), 6.79 (d, 1H, *J=* 15.9 Hz), 4.39 (d, 2H, *J=*5.7 Hz), 3.00 (s, 3H).
ESI [M+H]⁺: 428.0.

### Example 21: N-(3,5-Difluoro-4-methanesulfonylamino-benzyl)-3-(2-morpholin-4-yl-6-trifluoromethyl-pyridin-3-yl)-acrylamide

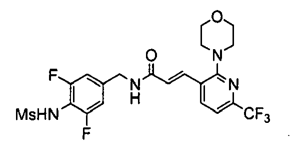

N-(4-Aminomethyl-2,6-difluoro-phenyl)-methanesulfonamide, HC1 salt (50mg, 0.22mmol) was reacted with 3-(2-Morpholin-4-yl-6-trifluoromethyl-pyridin-3-yl)-acrylic acid (55mg) to give N-(3,5-Difluoro-4-methanesulfonylamino-benzyl)-3-(2-morpholin-4-yl-6-trifluoromethyl-pyridin-3-yl)-acrylamide (37mg, 39%) after purification by column chromatography (Hex/EtOAc = 1/2).
¹H NMR(300MHz, CDCl₃): δ 7.78(d, 1H, *J* =8.1Hz), 7.75(d, 1H, *J* =15.3Hz), 7.25(d, 1H, *J* =8.1Hz), 6.98(d, 2H, *J* =8.4Hz), 6.46(d, 1H, *J* =15.3Hz), 6.15(s, 1H), 6.10(t, 1H), 4.38(d, 2H, *J*=6.6Hz), 3.85(m, 4H), 3.35(m, 4H), 3.21(s, 3H)
ESI [M+H]⁺: 521.1.

### Example 22: N-(3-Fluoro-4-methanesulfonylamino-5-trifluoromethyl-benzyl)-3-(2-morpholin-4-yl-6-trifluoromethyl-pyridin-3-yl)-acrylamide

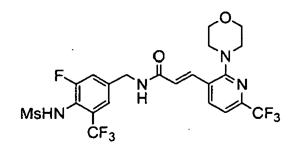

N-(4-Aminomethyl-2-fluoro-6-trifluoromethyl-phenyl)-methanesulfonamide, HCl salt (80mg, 0.25mmol) was reacted with 3-(2-Morpholin-4-yl-6-trifluoromethyl-pyridin-3-yl)-acrylic acid (76mg) to give N-(3-Fluoro-4-methanesulfonylamino-5-trifluoromethylbenzyl)-3-(2-morpholin-4-yl-6-trifluoromethyl-pyridin-3-yl)-acrylamide (56mg, 39%) after purification by column chromatography (Hex/EtOAc = 1/2).
¹H NMR(300MHz, CDCl₃): δ 8.71(s, 1H), 8.21(t, 1H), 7.65(d, 1H, *J* =7.8Hz), 7.49(d, 1H, *J* =15.6Hz), 7.29(s, 1H), 7.22(d, 1H, *J* =9.9Hz), 7.07(d, 1H, *J* =7.8Hz), 6.48(d, 1H, J=15.6Hz), 4.36(d, 2H, *J*=6.0Hz), 3.67(m, 4H), 3.18(m, 4H), 3.02(s, 3H)
ESI [M+H]⁺: 571.

### Example 23: N-(3-Ethynyl-5-fluoro-4-methanesulfonylamino-benzyl)-3-(6-morpholin-4-yl-pyridin-3-yl)-acrylamide

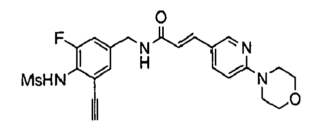

### Step 1: 3-(6-Morpholin-4-yl-pyridin-3-yl)-acrylic acid methyl ester

3-(6-Bromo-pyridin-3-yl)-acrylic acid methyl ester (100 mg, 0.413 mmol) was added to piperidine (1 ml) and the mixture was stirred for 1.5 hrs at room temperature. The reaction mixture was diluted with EtOAc and then washed three times with H₂O brine, dried. Na₂SO₄, filtered and concentrated under reduced pressure. The obtained residue was column-chromatographed to yield 3-(6-Morpholin-4-yl-pyridin-3-yl)-acrylic acid methyl ester (40 mg, 21 %).
¹H NMR (300 MHz, CDCl₃): δ 8.27 (d, 1H, *J =* 2.4 Hz), 7.68 (dd, 1H, *J* = 6.0, 2.4 Hz), 7.59 (d, 1H, *J* = 15.9 Hz), 6.62 (d, 1H, *J* = 9.3 Hz), 6.25 (d, 1H, *J=* 15.3 Hz), 3.82 - 3.78 (m, 7H), 3.61 - 3.57 (m, 4H).

### Step 2: N-(3-Ethynyl-5-fluoro-4-methanesulfonylamino-benzyl)-3-(6-morpholin-4-yl-pyridin-3-yl)-acrylamide

N-(4-Aminomethyl-2-ethynyl-6-fluoro-phenyl)-methanesulfonamide HCl salt (20 mg, 0.072 mmol) was suspended in THF and treated with triethylamine (11 mg, 0.108 mmol) and then the resulting mixture was stirred for 10mins. 3-(6-Morpholin-4-yl-pyridin-3-yl)-acrylic acid (17 mg, 0.072 mmol) was added to the reaction mixture followed by DMTMM (22 mg, 0.08 mmol) after 10 mins. The resulting mixture was stirred overnight at ambient temperature and then diluted with EtOAc. The resulting *s*olution was washed successively with water, sat'd NaHC0₃ (x2), brine, and then dried over anh. Na₂SO₄, filtered and concentrated under reduced pressure. The, crude residue was recrystallized (CH₂Cl₂) to yield the title compound (8 mg, 24 %).
¹H NMR (300 MHz, DMSO-d⁶): δ 9.39 (s, 1H), 8.52 (t, 1H, *J*= 5.4 Hz), 8.24 (s, 1H), 7.74 (d, 1H, *J* = 8.7 Hz), 7.34 (d, 1H, *J =* 15.9 Hz), 7.22-7.19 (m, 2H), 6.84 (d, 1H, *J* = 9.3 Hz), 6.45 (d, 1H, *J* = 15.9 Hz), 4.46 (s, 1H), 4.31 (d, 2H, *J =* 5.7 Hz), 3.63 -3.62 (m, 4H), 3.48 - 3.47(m, 4H), 3.01 (s, 3 H).
ESI [M+H]⁺: 459.

### Example 24: N-(3-Ethynyl-5-fluoro-4-methanesulfonylamino-benzyl)-3-(6'-trifluoromethyl-3,4,5,6-tetrahydro-2H-[1,2']bipyridinyl-3'-yl)-acrylamide

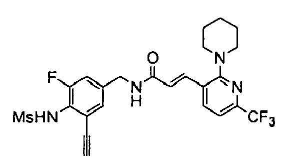

### Step 1: Synthesis of 3-(6'-Trifluoromethyl-3,4,5,6-tetrahydro-2H-[1,2']bipyridinyl-3'-yl)-acrylic acid

3-(6'-Trifluoromethyl-3,4,5,6-tetrahydro-2H-[1,2']bipyridinyl-3'-yl)-acrylic acid (720mg) was prepared by similar procedure as described in the previous from 2-chloro-6-trifluoromethylnicotinic acid in an overall yield of 54%.
¹H NMR(300MHz, CDCl₃): δ 7.81(m, 2H), 7.18(d, 1H, *J* =7.5Hz), 6.45(d, 1H, *J* =16.2Hz), 3.31(m, 4H), 1.72(m, 6H)

### Step 2: Synthesis of N-(3-Ethynyl-5-fluoro-4-methanesulfonylamino-benzyl)-3-(6'-trifluoromethyl-3,4,5,6-tetrahydro-2H-[1,2']bipyridinyl-3'-yl)-acrylamide

N-(4-Aminomethyl-2-ethynyl-6-fluoro-phenyl)-methanesulfonamide HCl salt (139mg, 0.50mmol) was reacted with 3-(6'-Trifluoromethyl-3,4,5,6-tetrahydro-2H-[1,2']bipyridinyl-3'-yl)-acrylic acid (150mg) to give N-(3-Ethynyl-5-fluoro-4-methanesulfonylamino-benzyl)-3-(6'-trifluoromethyl-3,4,5,6-tetrahydro-2H-[1,2']bipyridinyl-3'-yl)-acrylamide (132mg, 50%) after purification by column chromatography (Hex/EtOAc = 1/1).
¹H NMR(300MHz, CDCl₃): δ 7.72(m, 2H), 7.30(s, 1H), 7.16(m, 2H), 6.44(s, 1H), 6.43(d, 1H, *J* =15.6Hz), 6.10(s, 1H), 4.53(d, 2H, *J* =6.0Hz), 3.48(s, 1H), 3.30(m, 4H), 3.26(s, 3H), 1.65(m, 6H)
ESI [M+H]⁺: 525.

### Example 25: N-(3-Fluoro-4-methanesulfonylamino-benzyl)-3-(6-pyrrolidin-1-yl-pyridin-3-yl)-acrylamide

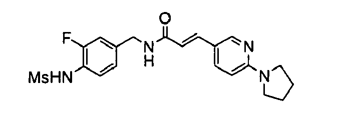

3-(6-Chloro-pyridin-3-yl)-N-(3-fluoro-4-methanesulfonylamino-benzyl)-acrylamide (15 mg, 0.413 mmol) was added to pyrrolidine (0.5 ml) and the mixture was stirred for 12 hrs at 90 °C. The resulting residue was dissolved in EtOAc, then washed three times with H₂O, and neutralized with 1N HCl to pH 5~7. The resulting solution was washed with brine, and then dried over anhyd. Na₂SO₄, filtered and concentrated under reduced pressure. The crude residue was recrystallized (CH₂Cl₂) to yield the title compound (4 mg, 24 %).
¹H NMR (300 MHz, DMSO-d⁶): δ, 8.41 (t, 1H, *J* = 6.0 Hz), 8.15 (s, 1H), 7.65 (d, 1H, *J* = 8.7 Hz), 7.29 (d, 1H, *J* = 16.8 Hz), 7.26 (d, 1H, *J =* 16.5 Hz), 7.10 (d, 1H, *J* = 11.7 Hz), 7.02 (d, 1H, *J* = 7.5 Hz), 6.43 (d, 1H, *J* = 9.3 Hz), 6.36 (d, 1H, *J* = 15.6 Hz), 4.29 (d, 2H, *J* = 5.7 Hz), 3.85 -3.75 (m, 4H), 2.92 (s, 3H), 3.48 - 3.47(m, 4H).
ESI [M+H]⁺: 419.

### Example 26: N-(3-Fluoro-4-methanesulfonylamino-benzyl)-3-(6'-trifluoromethyl-3,4,5,6-tetrahydro-2H-[1,2']bipyridinyl-3'-yl)-acrylamide

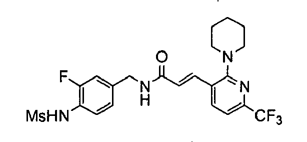

N-(4-Aminomethyl-2-fluoro-phenyl)-methanesulfonamide, HCl salt (127mg, 0.50mmol) was reacted with 3-(6'-Trifluoromethyl-3,4,5,6-tetrahydro-2H-[1,2']bipyridinyl-3'-yl)-acrylic acid (150mg) to give N-(3-Fluoro-4-methanesulfonylamino-benzyl)-3-(6'-trifluoromethyl-3,4,5,6-tetrahydro-2H-[1,2']bipyridinyl-3'-yl)-acrylamide (175mg, 70%) after purification by column chromatography (Hex/EtOAc = 1/1).
¹H NMR(300MHz, CDCl₃): δ 7.70(m, 2H), 7.50(t, 1H, *J* =8.1Hz), 7.12(m, 3H), 6.65(s, 1H), 6.45(d, 1H, *J* =15.6Hz), 6.14(t, 1H), 4.54(d, 2H, *J* =6.0Hz), 3.29(m, 4H), 3.02(s, 3H), 1.70(m, 6H)
ESI [M+H]⁺: 501.

### Example 27: N-(3-Fluoro-4-methanesulfonylamino-5-methyl-benzyl)-3-(2-morpholin-4-yl-6-trifluoromethyl-pyridin-3-yl)-propionamide

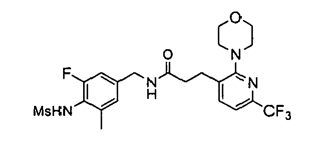

A suspension of N-(3-fluoro-4-methanesulfonylamino-5-methyl-benzyl)-3-(2-morpholin-4-yl-6-trifluoromethyl-pyridin-3-yl)-acrylamide (32mg, 0.069mmol) and 10% Pd/C (3mg) in MeOH was reacted under hydrogen of 40 psi pressure. The reaction mixture was filtered through celite and then concentrated under reduced pressure to give N-(3-fluoro-4-methanesulfonylamino-5-methyl-benzyl)-3-(2-morpholin-4-yl-6-trifluoromethyl-pyridin-3-yl)-propionamide (22mg, 69%).
¹H NMR(300MHz, CDCl₃): δ 7.61(d, 1H, *J* =6.9Hz), 7.27(d, 1H, *J* =8.4Hz), 7.10(m, 2H), 6.51 (s, 1H), 6.21(s, 1H), 4.42(d, 2H, *J* =5.1Hz), 3.86(m, 4H), 3.20(m, 4H), 3.07(m, 2H), 3.03(s, 3H), 2.63(m, 2H), 2.22(d, 3H, *J*=2.4Hz)
ESI [M+H]⁺: 519.

### Example 28: 2-(2-Bromo-6-tert-butyl-pyridin-3-yloxy)-N-(3-fluoro-4-methanesulfonylamino-benzyl)-acetamide

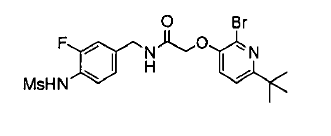

3-fluoro-4-methanesulfonylaminobenzylamine hydrochloride(72mg, 0.25mmol) was reacted with (2-Bromo-6-tert-butyl-pyridin-3-yloxy)-acetic acid (64mg), DMTMM(1.2eq, 83mg) and NEt₃(2.5eq, 90µl) in THF to give the title compound (71mg, 58.2%) after purification by column chromatography (Hex/EtOAc = 1/1).
¹H NMR(300MHz, CDCl₃): δ 7.56(m, 1H) 7.24(m, 2H) 7.11(m, 3H) 6.48(bs, 1H) 4.58 (s, 2H) 4.55(d, 2H, J=6Hz) 3.03(s, 3H) 1.33(s, 9H)
ESI [M+H]⁺: 488.

### Example 29: 2-(6-tert-Butyl-pyridin-3-yloxy)-N-(3-fluoro-4-methanesulfonylamino-benzyl)-acetamide

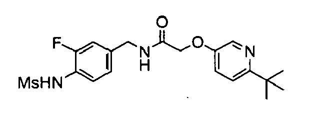

(3-fluoro-4-methanesulfonylaminobenzylamine hydrochloride(158mg, 0.62mmol) was reacted with (6-tert-Butyl-pyridin-3-yloxy)-acetic acid (130mg) DMTMM(1.2eq, 206mg) and NEt₃(2.5eq, 220µl) in THF to give title compound (165mg, 65%) after purification by column chromatography (Hex/EtOAc = 1/3).
¹H NMR(300MHz, CDCl₃): δ 8.33(d, 1H, *J*=0.6Hz) 7.38(m, 1H) 7.21(bs, 1H) 6.96(m, 2H) 6.88(m, 2H) 4.71 (s, 2H) 4.52(d, 2H, *J*=6Hz) 2.99(s, 3H) 1.47(s, 9H)
ESI [M+H]⁺: 409.

### Example 30: 2-(6-tert-Butyl-2-chloro-pyridin-3-yloxy)-N-(3-fluoro-4-methanesulfonylamino-benzyl)-acetamide

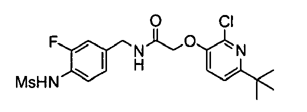

3-fluoro-4-methanesulfonylaminobenzylamine hydrochloride (21mg, 0.08mmo1) was reacted with (6-tert-Butyl-2-chloro-pyridin-3-yloxy)-acetic acid (1.0eq, 20mg) DMTMM(1.2eq, 27mg) and NMP(1.5eq, 14µl) in THF to give the title compound(10mg, 28.2%) after purification by column chromatography (Hex/EtOAc = 1/1).
¹H NMR(300MHz, CDCl₃): δ 7.55(m, 1H) 7.23(s, 1H) 7.16(s, 1H) 7.13(m, 2H) 7.10 (bs, 1H) 6.50(bs, 1H) 4.59(s, 2H) 4.54(d, 2H, *J*=6.3Hz) 3.03(s, 3H) 1.33(s, 9H)
ESI [M+H]⁺: 444.

### Example 31: N-(3-Fluoro-4-methanesulfonylamino-benzyl)-2-(2-hydroxymethyl-6-methyl-pyridin-3-yloxy)-acetamide

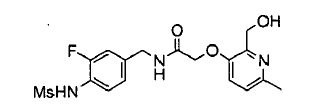

### Step 1: Synthesis of (2-Hydroxymethyl-6-methyl-pyridin-3-yloxy)-acetic acid

(2-Hydroxymethyl-6-methyl-pyridin-3-yloxy)-acetic acid (200mg) was prepared by similar procedure as described above from 2,6-lutidine- ²,3-diol in an overall yield of 51%.
¹H NMR(300MHz, DMSO-d₆): δ 7.24(d, 1H, *J* =8.1Hz), 7.10(d, 1H, *J* =8.1Hz), 4.73(s, 2H), 4.54(s, 2H), 2.40(s, 3H)

### Step 2: Synthesis of N-(3-Fluoro-4-methanesulfonylamino-benzyl)-2-(2-hydroxymethyl-6-methyl-pyridin-3-yloxy)-acetamide

N-(4-Aminomethyl-2-fluoro-phenyl)-methanesulfonamide HCl salt (270mg, 1.00mmol) was reacted with (2-Hydroxymethyl-6-methyl-pyridin-3-yloxy)-acetic acid (200mg) to give N-(3-Fluoro-4-methanesulfonylamino-benzyl)-2-(2-hydroxymethyl-6-methyl-pyridin-3-yloxy)-acetamide (350mg, 88%) after purification by column chromatography (Hex/EtOAc = 10/1).
¹H NMR(300MHz DMSO-d₆): δ 8.69(t, 1H), 7.29(m, 2H), 7.09(m, 3H), 5.10(t, 1H), 4.66(s, 2H), 4.60(d, 2H, *J* =5.4Hz), 4.31(d, 2H, *J*=6.3Hz), 2.99(s, 3H), 2.40(s, 3H)
ESI [M+H]⁺: 398.

### Example 32: N-(3-Fluoro-4-methanesulfonylamino-benzyl)-2-(6-trifluoromethyl-pyridin-3-yloxy)-acetamide

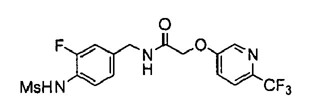

### Step 1: Synthesis of (6-Trifluoromethyl-pyridin-3-yloxy)-acetic acid

(6-Trifluoromethyl-pyridin-3-yloxy)-acetic acid (410mg) was prepared by similar procedure as described above from 2-trifluoromethyl-5-hydroxypyridine in an overall yield of 93%.
¹H NMR(300MHz, DMSO-d₆): δ 8.29(d, 1H, *J*=2.7Hz)*,* 7.75(d, 1H, *J*=8.7Hz), 7.34(dd, 1H, *J*=2.7 and 8.7Hz), 4.33(s, 2H)

### Step 2: Synthesis of N-(3-Fluoro-4-methanesulfonylamino-benzyl)-2-(6-trifluoromethyl-pyridin-3-yloxy)-acetamide

N-(4-Aminomethyl-2-fluoro-phenyl)-methanesulfonamide HCl salt (270mg, 1.00mmol) was reacted with (6-Trifluoromethyl-pyridin-3-yloxy)-acetic acid (221mg) to give N-(3-Fluoro-4-methanesulfonylamino-benzyl)-2-(6-trifluoromethyl-pyridin-3-yloxy)-acetamide (54mg, 88%) after recrystalization from Hex/EtOAc.
¹H NMR(300MHz DMSO-d₆): δ 9.52(s, 1H), 8.80(t, 1H), 8.49(d, 1H, *J* =2.7Hz), 7.87(d, 1H, *J* =9.0Hz), 7.60(dd, 1H, *J* =2.7 and 9.0Hz), 7.32(m, 1H), 7.11(m, 2H), 4.80(s, 2H), 4.33(d, 2H, *J*=6.3Hz), 2.99(s, 3H)
ESI [M+H]⁺: 422.

### Example 33: 2-(6-tert-Butyl-5-chloro-pyridin-3-yloxy)-N-(3-fluoro-4-methanesulfonylamino-benzyl)-acetamide

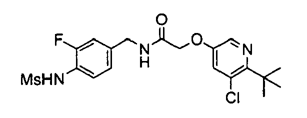

3-fluoro-4-methanesulfonylaminobenzylamine hydrochloride (192mg, 0.751minol) was reacted with (6-tert-Butyl-5-chloro-pyridin-3-yloxy)-acetic acid (183mg) DMTMM(1.1eq, 229mg) and NMP(1.2eq, 100µl) in THF to give the title compound (102mg, 30.6%) after purification by column chromatography (Hex/EtOAc = 1/1).
¹H NMR(300MHz, CDCl₃): δ 8.18(d, 1H, *J* =1.8Hz) 7.56(t, 1H) 7.10(m, 2H) 7.07(m, 1H) 6.71(bs, H) 6.50 (bs, 1H) 4.60(s, 2H) 4.54(d, 2H, J=6Hz) 3.03(s, 3H) 1.38(s, 9H)
ESI [M+H]⁺: 444.

### Example 34: 2-(6-tert-Butyl-pyridin-3-yloxy)-N-(3-ethynyl-5-fluoro-4-methanesulfonylamino-benzyl)-acetamide

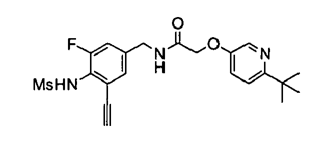

N-(4-Aminomethyl-2-ethynyl-6-fluoro-phenyl)-methanesulfonamide HCl salt (120mg, 0.43mmol) was reacted with (6-tert-Butyl-pyridin-3-yloxy)-acetic acid (90mg) DMTMM(1.1eq, 131mg) and NMP(1.2eq, 60µl) in THF to give the title compound (62mg, 33.3%) after purification by column chromatography (Hex/EtOAc = 2/3).
¹H NMR(300MHz, CDCl₃): δ 8.36(d, 1H, *J* =1.8Hz) 7.22(bs, 1H) 7.18(m, 1H) 7.06(m, 1H) 6.89(ts, 1H) 6.64 (s, 1H) 4.68(s, 2H) 4.51(d, 2H, *J* =6Hz) 3.46(s, 1H) 3.23(s, 3H) 1.47(s, 9H)
ESI [M+H]⁺: 434.

### Example 35: 2-(6-Chloro-pyridin-3-yloxy)-N-(3-ethynyl-5-fluoro-4-methanesulfonylamino-benzyl)-acetamide

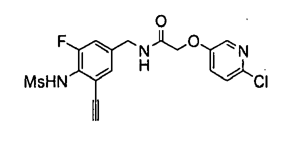

### Step 1: (6-Chloro-pyridin-3-yloxy)-acetic acid ethyl ester

To a suspension of 6-Chloro-pyridin-3-ol (1000 mg, 7.72 mmol) and Bromoethyl acetate (1933 mg, 11.57 mmol) in CH₃CN was added Cs₂CO₃ (3772 mg, 11.57 mmol). The mixture was stirred for overnight at room temperature. The reaction mixture was diluted with EtOAc, then washed three times with H₂O and brine, and then dried. MgSO₄, filtered and concentrated under reduced pressure. The obtained residue was column-chromatographed to yield the (6-Chloro-pyridin-3-yloxy)-acetic acid ethyl ester (1.5 g, 90%).
¹H-NMR (300MHz, CDCl₃): δ 8.07 (dd, 1H, *J* = 2.7, 0.9 Hz), 7.27 - 7.23 (m, 2H), 4.65 (s, 2H), 4.28 (q, 2H, *J =* 7.2 Hz), 1.30 (t, 3H, *J =* 7.2 Hz).

### Step 2: (6-Chloro-pyridin-3-yloxy)-acetic acid

(6-Chloro-pyridin-3-yloxy)-acetic acid ethyl ester (800 mg, 3.71 mmol) in THF was added to a solution of 0.5 N-LiOH (2 eq) and the mixture was stirred for 1.5 hrs at room temperature. The resulting residue was dissolved in H₂O then washed three times with Et₂O and neutralized with 1N HCl to pH 5-7. The solution was extracted three times with methylene chloride and then dried over anhyd. Na₂SO₄ and concentrated in vacuo to give (6-Chloro-pyridin-3-yloxy)-acetic acid (300 mg, 36 %).
¹H-NMR (300MHz, CDCl₃): δ 8.05 (d, 1H, *J=* 3.3 Hz), 7.43 - 7.35 (m, 2H), 4.74 (s, 2H).

### Step 3: 2-(6-Chloro-pyridin-3-yloxy)-N-(3-ethynyl-5-fluoro-4-methanesulfonylamino-benzyl)-acetamide

N-(4-Aminomethyl-2-ethynyl-6-fluoro-phenyl)-methanesulfonamide (74.3 mg, 0.266 mmol) was suspended in THF and treated with triethylamine (27 mg, 0.266 mmol) and then the resulting mixture was stirred for 10mins. (6-Chloro-pyridin-3-yloxy)-acetic acid (50 mg, 0.266 mmol) was added to the reaction mixture followed by DMTMM (62.5 mg, 0.266 mmol) after 10 mins. The resulting mixture was stirred overnight at ambient temperature and then diluted with EtOAc. The resulting solution was washed successively with water, sat'd NaHCO₃ (x3), and brine, and then dried over anh. MgSO₄, filtered and concentrated under reduced pressure. The crude residue was recrystallized (CH₂Cl₂) to yield the title compound (25 mg, 25 %).
¹H NMR (300 MHz, CDCl3): δ 8.13 (dd, 1H, *J*= 3.0, 0.9 Hz), 7.34 - 7.24 (m, 3H), 7.14 (dd, 1H, *J*= 10.5, 2.1 Hz), 6.90 (s, 1H, br), 6.44 (s, 1H), 4.58 (s, 2H), 4.53 (s, 1H), 4.50 (d, 2H, *J*= 6.3 Hz), 3.26 (s, 3H).
ESI [M+H]⁺: 412.

### Example 36: 3-(2-Bromo-6-tert-butyl-pyridin-3-yl)-N-(3-fluoro-4-methanesulfonylamino-benzyl)-acrylamide

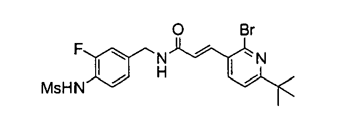

3-fluoro-4-methanesulfonylaminobenzylamine hydrochloride (116mg, 0.454mmol) was reacted with 3-(2-Bromo-6-tert-butyl-pyridin-3-yl)-acrylic acid (1.0eq, 129mg) DMTMM(1.0eq, 126mg) and NMP(1.2eq, 60µl) in THF to give the title compound (72mg, 32.8%) after purification by column chromatography (Hex/EtOAc = 1/1).
¹H NMR(300MHz, CDCl₃): δ 7.89(d, 1H, *J* =12.9Hz), 7.72(d, 1H, *J* =7.8Hz) 7.56(m, 1H) 7.29(d, 1H, *J* =8.4Hz) 7.15(m, 2H) 6.46(bs, 1H) 6.36(d, 1H, *J* =15.3Hz) 6.01(bs, 1H) 4.56(d, 2H, *J*=6.3Hz) 3.03(s, 3H) 1.35(s, 9H)

### Example 37: 3-(2-Bromo-6-tert-butyl-pyridin-3-yl)-N-(3-ethynyl-5-fluoro-4-methanesulfonylamino-benzyl)-acrylamide

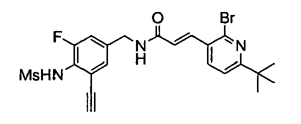

N-(4-Aminomethyl-2-ethynyl-6-fluoro-phenyl)-methanesulfonamide HCl salt (41.2mg, 0.148mmol) was reacted with 3-(2-Bromo-6-tert-butyl-pyridin-3-yl)-acrylic acid (1.0eq, 42mg) DMTMM(1.0eq, 41mg) and NMP(1.2eq, 20µl) in THF to give the title compound (42mg, 55.8%) after purification by column chromatography (Hex/EtOAc = 3/2).
¹H NMR(300MHz, CDCl₃): δ 7.90(d, 1H, *J* =15.9Hz), 7.73(d, 1H, *J* =7.5Hz) 7.31(m, 1H) 7.28(s, 1H), 7.17(dd, 1H, *J* =2.1Hz and 1.5Hz) 6.41(s, 1H), 6.37(d, 1H, *J*=15.7Hz) 6.09(bs, 1H), 4.53(d, 2H, *J* =6.0Hz), 3.48(s, 1H), 3.27(s, 3H) 1.35(s, 9H)

### Experimental example: Biological potency test

### 1. ⁴⁵Ca influx test

### 1) Separation of spinal dorsal root ganglia (DRG) in newborn rats and primary culture thereof

Neonatal (2-3 day old or younger than 2-3 day old) SD rats were put in ice for 5 minutes to anesthetize and disinfected with 70% ethanol. DRG of all part of spinal cord were dissected (Wood et al., 1988, J. Neurosci. 8, pp3208-3220) and collected in DME/F12 medium to which 1.2g/l sodium bicarbonate, 50mg/l gentamycin were added. The DRG were incubated sequentially at 37°C for 30 mins in 200 U/ml collagenase and 2.5mg/ml trypsin, separately. The ganglia were washed twice with DME/F12 medium supplemented with 10% horse serum, triturated through a fire-polished Pasteur pipette, filtered through Nitex 80 membrane to obtain single cell suspension and the suspension was washed once more. This was subjected to centrifugation, then resuspended in cell culture medium at certain level of cell density. As the cell culture medium, DME/F12 medium supplemented with 10% horse serum was diluted with identical medium conditioned by C6 glioma cells 2 days on a confluent monolayer (1:1), and NGF (Nerve Growth Factor) was added to adjust 200ng/ml as final concentration. After the cells were grown 2 days in medium where cytosine arabinoside (Ara-C, 100 µM) was added to kill dividing nonneuronal cells, medium was changed to one without Ara-C. The resuspended cells were plated at a density of 1500-2000 neurons/well onto Terasaki plates previously coated with 10 µg/ml poly-D-ornithine.

### 2) ⁴⁵Ca influx experiments

DRG nerve cells from the primary culture of 2 days were equilibrated by washing 4 times with HEPES (10mM, pH 7.4)-buffered Ca²⁺, Mg²⁺-free HBSS (H-HBSS). The solution in each well was removed from the individual well. Medium containing the test compound plus capsaicin (final concentration 0.5 µM) and ⁴⁵Ca (final concentration 10 µCi/ml) in H-HBSS was added to each well and incubated at room temperature for 10 mins. Terasaki plates were washed five times with H-HBSS and dried at room temperature. To each well, 0.3% SDS (10 µl) was added to elute ⁴⁵Ca. After the addition of scintillation cocktail of into each well, the amount of ⁴⁵Ca influx into neuron was measured by counting radioactivity. Antagonistic activities of test compounds against vanilloid receptor were calculated as percent of the inhibition of maximal response of capsaicin at a concentration of 0.5 µM and the results are given as IC₅₀.

**[Table 1] Results of Calcium Influx Test**

| **Examples** | **Antagonist** |
|---|---|
| | **Calcium Uptake Test (IC₅₀, µM)** |
| **1** | 1.3 |
| **2** | 1.3 |
| **3** | 0.52 |
| **4** | 0.22 |
| **5** | 1.5 |
| **6** | 0.30 |
| **7** | 1.0 |
| **8** | 0.42 |
| **9** | 0.40 |
| **10** | 0.28 |
| **11** | 0.29 |
| **12** | 0.26 |
| **13** | 3.5 |
| **14** | >10 |
| **15** | 5.9 |
| **16** | >10 |
| **17** | 0.57 |
| **18** | 0.13 |
| **19** | >10 |
| **20** | >10 |
| **21** | 1.1 |
| **22** | 3.3 |
| **23** | >10 |
| **24** | 0.34 |
| **25** | >10 |
| **26** | 1.1 |
| **27** | 2.9 |
| **28** | 1.6 |
| **29** | >10 |
| **30** | 3.8 |
| **31** | >10 |
| **32** | >10 |
| **33** | 1.0 |
| **34** | >10 |
| **35** | >10 |
| **36** | 0.18 |
| **37** | 0.059 |

### 2. Analgesic activity test: Mouse writhing test by inducing with phenyl-p-quinone

Male ICR mice (mean body weight 25g) were maintained in a controlled lighting environment (12 h on/ 12 h off) for experiment. Animals received an intraperitoneal injection of 0.3ml of the chemical irritant phenyl-p-quinone (dissolved in saline containing 5% ethanol to be a dose of 4.5mg/kg) and 6 mins later, the number of abdominal constrictions was counted in the subsequent 6 mins period. Animals (10 animals/group) received 0.2ml of test compounds solution in vehicle of ethanol/Tween 80/saline (10/10/80) intraperitoneally 30 mins before the injection of phenyl-p-quinone. In the case of oral administration, 0.2ml of test compounds solution in vehicle of ethanol/Tween 80/saline (5/5/90) were administered 54 mins prior to the 0.2ml of 0.02% phenyl-p-quinone injection. A reduction in the number of writhes responding to the test drug compound relative to the number responding in saline control group was considered to be indicative of an analgesic effect. Analgesic effect was calculated by % inhibition equation (% inhibition=(C-T)/C x 100), wherein C and T represent the number of writhes in control and compound-treated group, respectively. Most examples of the present invention having good in vitro activities, were tested at various doses (0.1 to 3 mg/kg) and resulted in good analgesic effect from 30 to 64% inhibition.

### Industrial Applicability

As explained above, the compound according to the present invention is useful to prevent or to treat pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis, and pancreatitis.
More specifically, the compound according to the present invention is useful to preventing and treating of pain, which is or which is associated with a condition selected from the group consisting of osteoarthritis, rheumatoid arthritis, ankylosing spondylitis, diabetic neuropathic pain, post-operative pain, dental pain, non-inflammatory musculoskeletal pain (including fibromyalgia, myofascial pain syndrome and back pain), migraine, and other types of headaches.

## Claims

1. A compound of the formula (I), an isomer thereof, or a pharmaceutically acceptable salt thereof: 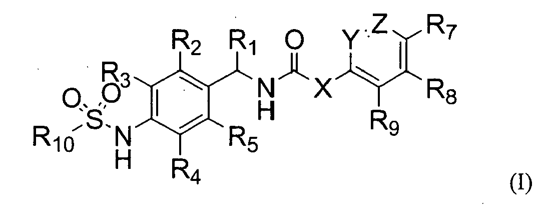 wherein,
X is CR₁₁=CR₁₂, CHR₁₁CHR₁₂, 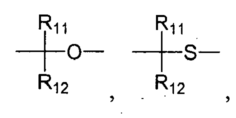 or C=C, wherein, R₁₁ and R₁₂, if present, are independently hydrogen, halogen, or C1-C5 alkyl;
Y and Z are independently CH, CR₆, or N, such that at least one of Y and Z is N;
R₁ is hydrogen or C1-C5 alkyl;
R₂, R₃, R₄, and R₅ are independently hydrogen, halogen, nitro, cyano, C1-C5 alkyl, C1-C5 alkoxy, halo (C1-C5) alkyl, C2-C5 alkenyl, C2-C5 alkynyl, carboxy, C1-C5 alkoxycarbonyl, or C1-C5 alkylthio;
R₆, R₇, R₈, and R₉ are independently hydrogen, hydroxy, halogen, nitro, carboxy, C1-C5 alkyl, C1-C5 alkoxy, hydroxy (C1-C5) alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C5 alkyl)amino, C3-C6 cycloalkyl which may be unsubstituted or substituted with one or more C1-C3 alkyl groups, pyrrolidinyl, phenyl, or morpholinyl, wherein the phenyl may be unsubstituted or substituted with one or more substituents selected from halogen, C1-C5 alkyl, and halo (C1-C5) alkyl; and
R₁₀ is C1-C5 alkyl, halo (C1-C5) alkyl, or C2-C5 alkenyl.

2. The compound according to claim 1, an isomer, or a pharmaceutically acceptable salt thereof;
wherein,
X is CR₁₁=CR₁₂, CHR₁₁CHR₁₂, 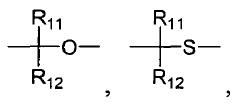 or C=C, wherein, R₁₁ and R₁₂, if present, are independently hydrogen, halogen, or C1-C3 alkyl;
R₁ is hydrogen or C1-C3 alkyl;
R₂, R₃, R₄, and R₅ are independently hydrogen, halogen, nitro, cyano, methyl, ethyl, ethenyl, ethynyl, trifluoromethyl, methoxy, ethoxy, or methoxycarbonyl;
R₆, R₇, R₈, and R₉ are independently hydrogen, hydroxy, halogen, nitro, carboxy, C1-C5 alkyl, C1-C5 alkoxy, hydroxy (C1-C5) alkyl, C2-C5 alkenyl, C2-C5 alkynyl, halo (C1-C5) alkyl, halo (C1-C5) alkoxy, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C3 alkyl)amino, C3-C6 cycloalkyl which may be unsubstituted or substituted with one or more methyl groups, pyrrolidinyl, phenyl, or morpholinyl, wherein the phenyl may be unsubstituted or substituted with one or more substituents selected from halogen, C1-C5 alkyl, and halo (C1-C5) alkyl; and
R₁₀ is C1-C3 alkyl or C2-C3 alkenyl.

3. A compound of the formula (II), an isomer, or a pharmaceutically acceptable salt thereof; 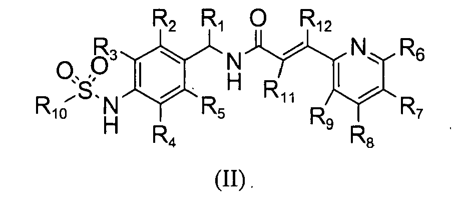 wherein,
R₁ is hydrogen or C1-C5 alkyl;
R₂, R₃, R₄, and R₅ are independently hydrogen, halogen, nitro, cyano, C1-C5 alkyl, C1-C5 alkoxy, halo (C1-C5) alkyl, C2-C5 alkenyl, C2-C5 alkynyl, carboxy, C1-C5 alkoxycarbonyl, or C1-C5 alkylthio;
R₆, R₇, R₈, and R₉ are independently hydrogen, hydroxy, halogen, nitro, carboxy, C1-C5 alkyl, C1-C5 alkoxy, C2-C5 alkenyl, C2-C5 alkynyl, halo (C1-C5) alkyl, halo (C1-C5) alkoxy, hydroxy (C1-C5) alkyl, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C5 alkyl)amino, C3-C6 cycloalkyl which may be unsubstituted or substituted with one or more C1-C3 alkyl groups, pyrrolidinyl, phenyl, or morpholinyl, wherein the phenyl may be unsubstituted or substituted with one or more substituents selected from halogen, C1-C5 alkyl, and halo (C1-C5) alkyl;
R₁₀ is C1-C5 alkyl, halo (C1-C5) alkyl, or C2-C5 alkenyl; and
R₁₁ and R₁₂ are independently hydrogen, C1-C5 alkyl, or halogen.

4. A compound of the formula (III), an isomer, or a pharmaceutically acceptable salt thereof; 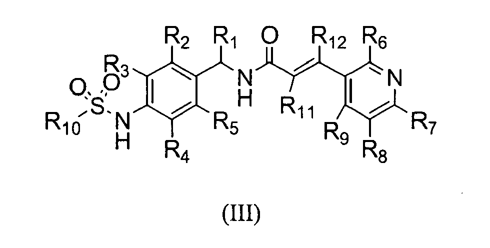 wherein,
R₁ is hydrogen or C1-C5 alkyl;
R₂, R₃, R₄, and R₅ are independently hydrogen, halogen, nitro, cyano, C1-C5 alkyl, C1-C5 alkoxy, halo (C1-C5) alkyl, C2-C5 alkenyl, C2-C5 alkynyl, carboxy, C1-C5 alkoxycarbonyl, or C1-C5 alkylthio;
R₆, R₇, R₈, and R₉ are independently hydrogen, hydroxy, halogen, nitro, carboxy, C1-C5 alkyl, C1-C5 alkoxy, C2-C5 alkenyl, C2-C5 alkynyl, halo (C1-C5) alkyl, halo (C1-C5) alkoxy, hydroxy (C1-C5) alkyl, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C5 alkyl)amino, C3-C6 cycloalkyl which may be unsubstituted or substituted with one or more C1-C3 alkyl groups, pyrrolidinyl, phenyl, or morpholinyl, wherein the phenyl may be unsubstituted or substituted with one or more substituents selected from halogen, C1-C5 alkyl, and halo (C1-C5) alkyl;
R₁₀ is C1-C5 alkyl, halo (C1-C5) alkyl, or C2-C5 alkenyl; and
R₁₁ and R₁₂ are independently hydrogen, C1-C5 alkyl, or halogen.

5. The compound according to anyone of claims 1 to 4, an isomer, or a pharmaceutically acceptable salt thereof;
wherein,
R₁ is hydrogen, methyl, or ethyl;
R₂, R₃, R₄, and R₅ are independently hydrogen, fluoro, chloro, bromo, nitro, cyano, methyl, ethyl, ethenyl, ethynyl, trifluoromethyl, methoxy, ethoxy, or methoxycarbonyl; R₆, if present, is hydrogen, hydroxy, fluoro, bromo, chloro, hydroxymethyl, C1-C5 alkyl, C1-C5 alkoxy, C2-C5 alkenyl, C2-C5 alkynyl, halo (C1-C5) alkyl, halo (C1-C5) alkoxy, C2-C5 alkenyloxy, C1-C5 alkoxy (C1-C5) alkoxy, C1-C5 alkoxy (C1-C5) alkoxy (C1-C5) alkyl, di(C1-C3 alkyl)amino, C1-C3 alkylpiperazinyl, piperidyl, pyrrolidinyl, halophenyl, phenyl, or morpholinyl;
R₇ is C1-C5 alkyl, halo (C1-C4) alkyl, halogen, piperidyl, morpholinyl, pyrrolidinyl, C3-C6 cycloalkyl which may be unsubstituted or substituted with one or more methyl groups, C2-C5 alkenyl;
R₈ and R₉ are independently hydrogen, halogen, or trifluoromethyl;
R₁₀ is C1-C5 alkyl, halo (C1-C5) alkyl, or C2-C5 alkenyl; and
R₁₁ and R₁₂, if present, are independently hydrogen, or methyl.

6. The compound according to anyone of claims 1 to. 5, an isomer, or a pharmaceutically acceptable salt thereof;
wherein,
R₁, and R₂ are hydrogen;
R₃ is hydrogen, fluoro, chloro, bromo, cyano, methyl, ethyl, ethenyl, ethynyl, or trifluoromethyl;
R₄ and R₅ are independently hydrogen, fluoro, chloro, cyano, methyl, ethyl, or trifluoromethyl;
R₆, if present, is hydrogen, hydroxy, fluoro, bromo, chloro, methyl, hydroxymethyl, methoxy, trifluoromethyl, di(C1-C3 alkyl)amino, alkylpiperazinyl, piperidyl, pyrrolidinyl, halophenyl, phenyl, or morpholinyl;
R₇ is methyl, isopropyl, t-butyl, trifloromethyl, chloro, bromo, cyclopropyl which may be unsubstituted or substituted with one or two methyl groups, piperidyl, pyrrolidinyl, or morpholinyl;
R₈ is hydrogen;
R₉ is hydrogen or trifluoromethyl;
R₁₀ is C1-C5 alkyl, halo (C1-C5) alkyl, or C2-C5 alkenyl; and
R₁₁ and R₁₂, if present, are independently hydrogen or methyl.

7. The compound according to anyone of claims 1 to 6, an isomer, or a pharmaceutically acceptable salt thereof;
wherein,
R₁, R₂, and R₅ are hydrogen;
R₃ is hydrogen, fluoro, chloro, cyano, methyl, ethenyl, ethynyl, or trifluoromethyl;
R₄ is hydrogen, fluoro, chloro, cyano, methyl, ethyl, or trifluoromethyl;
R₆, if present, is hydrogen, hydroxy, fluoro, bromo, chloro, methyl, hydroxymethyl, methoxy, trifluoromethyl, diethylamino, piperidyl, pyrrolidinyl, trifluorophenyl, phenyl, or morpholinyl;
R₇ is methyl, isopropyl, t-butyl, trifluoromethyl, chloro, bromo, cyclopropyl, methylcyclopropyl, piperidyl, pyrrolidinyl, or morpholinyl;
R₈ is hydrogen;
R₁₁ and R₁₂, if present, are hydrogen;
R₉ is hydrogen or trifluoromethyl; and
R₁₀ is methyl.

8. The compound according to anyone of claims 1 to 5, an isomer, or a pharmaceutically acceptable salt thereof;
wherein,
R₁ is hydrogen or methyl;
R₂ is hydrogen;
R₃ is hydrogen, fluoro, chloro, bromo, cyano, methyl, ethyl, ethenyl, ethynyl, or trifluoromethyl;
R₄ and R₅ are independently hydrogen, fluoro, chloro, cyano, methyl, ethyl, or trifluoromethyl;
R₆, if present, is hydrogen, fluoro, chloro, bromo, methyl, methoxy, piperidyl, or morpholinyl;
R₇ is isopropyl, t-butyl, or trifluoromethyl;
R₈ is hydrogen;
R₁₁ and R₁₂, if present, are hydrogen;
R₉ is hydrogen or trifluoromethyl; and
R₁₀ is methyl.

9. The compound according to anyone of claims 1 to 8, an isomer, or a pharmaceutically acceptable salt thereof, wherein the compound is selected from the group consisting of
3-(6-tert-Butyl-pyridin-3-yl)-N-(3-chloro-4-methanesulfonylamino-benzyl)-acrylamide,
3-(6-tert-Butyl-pyridin-3-yl)-N-(3-fluoro-4-methanesulfonylamino-benzyl)-acrylamide,
3-(6-tert-Butyl-pyridin-3-yl)-N-(3-ethynyl-5-fluoro-4-methanesulfonylamino-benzyl)-acrylamide,
3-(6-tert-Butyl-pyridin-3-yl)-N-(4-methanesulfonylamino-3-vinyl-benzyl)-acrylamide,
N-(3-Fluoro-4-methanesulfonylamino-benzyl)-3-(2-morpholin-4-yl-6-trifluoromethylpyridin-3-yl)-acrylamide,
N-(4-Methanesulfonylamino-3-vinyl-benzyl)-3-(2-morpholin-4-yl-6-trifluoromethyl-pyridin-3-yl)-acrylamide,
N-(3-Chloro-4-methanesulfonylamino-benzyl)-3-(2-morpholin-4-yl-6-trifluoromethyl-pyridin-3-yl)-acrylamide,
N-(3-Fluoro-4-methanesulfonylamino-5-methyl-benzyl)-3-(2-morpholin-4-yl-6-trifluoromethyl-pyridin-3-yl)-acrylamide,
3-(6-tert-Butyl-2-methoxy-pyridin-3-yl)-N-(3-fluoro-4-methanesulfonylamino-benzyl)-acrylamide,
3-(6-tert-Butyl-pyridin-3-yl)-N-(3-fluoro-4-methanesulfonylamino-5-vinyl-benzyl)-acrylamide,
3-(6-tert-Butyl-2-methoxy-pyridin-3-yl)-N-(3-ethynyl-5-fluoro-4-methanesulfonylamino-benzyl)-acrylamide,
N-(3-Ethynyl-5-fluoro-4-methanesulfonylamino-benzyl)-3-(2-morpholin-4-yl-6-trifluoromethyl-pyridin-3-yl)-acrylamide,
3-(6-tert-Butyl-4-trifluoromethyl-pyridin-3-yl)-N-(3-fluoro-4-methanesulfonylamino-benzyl)-acrylamide,
3-(6-tert-Butyl-4-trifluoromethyl-pyridin-3-yl)-N-(3-ethynyl-5-fluoro-4-methanesulfonylamino-benzyl)-acrylamide,
N-(3,5-Difluoro-4-methanesulfonylamino-benzyl)-3-(2-morpholin-4-yl-6-trifluoromethyl-pyridin-3-yl)-acrylamide,
N-(3-Ethynyl-5-fluoro-4-methanesulfonylamino-benzyl)-3-(6'-trifluoromethyl-3,4,5,6-tetrahydro-2H-[1,2']bipyridinyl-3'-yl)-acrylamide,
3-(2-Bromo-6-tert-butyl-pyridin-3-yl)-N-(3-fluoro-4-methanesulfonylamino-benzyl)-acrylamide,
3-(2-Bromo-6-tert-butyl-pyridin-3-yl)-N-(3-ethynyl-5-fluoro-4-methanesulfonylamino-benzyl)-acrylamide, and
N-(3-Fluoro-4-methanesulfonylamino-benzyl)-3-(6'-trifluoromethyl-3,4,5,6-tetrahydro-2H-[1,2']bipyridinyl-3'-yl)-acrylamide.

10. The compound according to anyone of claims 1 to 8, an isomer, or a pharmaceutically acceptable salt thereof, wherein the compound is selected from the group consisting of;
3-(6-tert-Butyl-pyridin-3-yl)-N-(3-ethynyl-5-fluoro-4-methanesulfonylamino-benzyl)-acrylamide,
3-(6-tert-Butyl-pyridin-3-yl)-N-(4-methanesulfonylamino-3--benzyl)-acrylamide,
N-(4-Methanesulfonylamino-3-vinyl-benzyl)-3-(2-morpholin-4-yl-6-trifluoromethyl-pyridin-3-yl)-acrylamide,
N-(3-Fluoro-4-methanesulfonylamino-5-methyl-benzyl)-3-(2-morpholin-4-yl-6-trifluoromethyl-pyridin-3-yl)-acrylamide,
3-(6-tert-Butyl-2-methoxy-pyridin-3-yl)-N-(3-fluoro-4-methanesulfonylamino-benzyl)-acrylamide,
3-(6-tert-Butyl-pyridin-3-yl)-N-(3-fluoro-4-methanesulfonylamino-5-vinyl-benzyl)-acrylamide,
3-(6-tert-Butyl-2-methoxy-pyridin-3-yl)-N-(3-ethynyl-5-fluoro-4-methanesulfonylamino-benzyl)-acrylamide,
N-(3-Ethynyl-5-fluoro-4-methanesulfonylamino-benzyl)-3-(2-morpholin-4-yl-6-trifluoromethyl-pyridin-3-yl)-acrylamide,
3-(6-tert-Butyl-4-trifluoromethyl-pyridin-3-yl)-N-(3-fluoro-4-methanesulfonylamino-benzyl)-acrylamide,
3-(6-tert-Butyl-4-trifluoromethyl-pyridin-3-yl)-N-(3-ethynyl-5-fluoro-4-methanesulfonylamino-benzyl)-acrylamide,
N-(3-Ethynyl-5-fluoro-4-methanesulfonylamino-benzyl)-3-(6'-trifluoromethyl-3,4,5,6-tetrahydro-2H-[1,2']bipyridinyl-3'-yl)-acrylamide,
3-(2-Bromo-6- tert-butyl-pyridin-3-yl)-N-(3-fluoro-4-methanesulfonylamino-benzyl)-acrylamide, and
3-(2-Bromo-6-tert-butyl-pyridin-3-yl)-N-(3-ethynyl-5-fluoro-4-methanesulfonylamino-benzyl)-acrylamide.

11. A compound of the formula (IV), an isomer, or a pharmaceutically acceptable salt thereof; 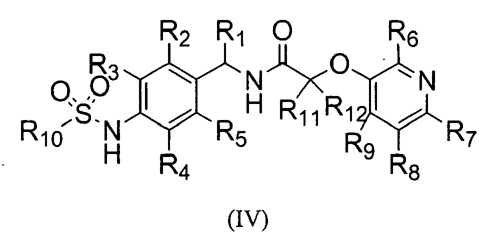 wherein,
R₁ is hydrogen, methyl, or ethyl;
R₂, R₃, R₄, and R₅ are independently hydrogen, fluoro, chloro, bromo, nitro, cyano, methyl, ethyl, ethenyl, ethynyl, trifluoromethyl, methoxy, ethoxy, hydroxy, or methoxycarbonyl;
R₆ is hydrogen, fluoro, bromo, chloro, hydroxymethyl, C1-C5 alkyl, C1-C5 alkoxy, C2-C5 alkenyl, C2-C5 alkynyl, halo (C1-C5) alkyl, halo (C1-C5) alkoxy, C2-C5 alkenyloxy, C1-C5 alkoxy (C1-C5) alkoxy, piperidyl, or morpholinyl;
R₇ is C1-C5 alkyl, halo (C1-C4) alkyl, halogen, morpholinyl, C3-C6 cycloalkyl, C2-C5 alkenyl, or nitro;
R₈ and R₉ are hydrogen;
R₁₀ is C1-C5 alkyl, halo (C1-C5) alkyl, or C2-C5 alkenyl; and
R₁₁ and R₁₂ are independently hydrogen, halogen, or methyl.

12. The compound according to claim 11, an isomer, or a pharmaceutically acceptable salt thereof;
wherein,
R₁, R₂, R₅, R₈, and R₉ are hydrogen;
R₃ and R₄ are independently hydrogen, fluoro, chloro, cyano, methyl, ethenyl, ethynyl, or trifluoromethyl;
R₆ is hydrogen, fluoro, bromo, chloro, hydroxymethyl, methoxy, morpholinyl, or trifluoromethyl;
R₇ is t-butyl, trifloromethyl, methyl, chloro, bromo, nitro, or morpholinyl; and
R₁₀ is methyl or ethenyl.

13. The compound according to claims 11 or 12, an isomer, or a pharmaceutically acceptable salt thereof;
wherein,
R₁, R₂, R₅, R₈, and R₉ are hydrogen;
R₃ and R₄ are independently hydrogen, fluoro, chloro, cyano, methyl, ethenyl, ethynyl, or trifluoromethyl;
R₆ is hydrogen, fluoro, bromo, chloro, hydroxymethyl, methoxy, morpholinyl, or trifluoromethyl;
R₇ is t-butyl, trifloromethyl, methyl, chloro, bromo, nitro, or morpholinyl; and
R₁₀ is methyl.

14. A compound of the formula (V), an isomer, or a pharmaceutically acceptable salt thereof; 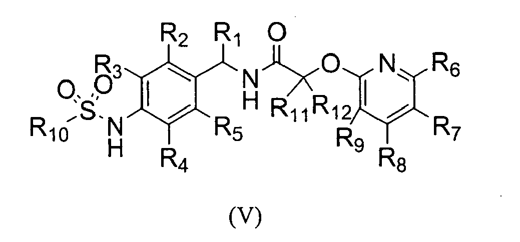 wherein,
R₁ is hydrogen, methyl, or ethyl;
R₂, R₃, R₄, and R₅ are independently hydrogen, fluoro, chloro, bromo, nitro, cyano, methyl, ethyl, ethenyl, ethynyl, trifluoromethyl, methoxy, ethoxy, or methoxycarbonyl;
R₆, R₇, R₈ and R₉ are independently hydrogen, fluoro, bromo, chloro, hydroxymethyl, C1-C5 alkyl, C1-C5 alkoxy, C2-C5 alkenyl, C2-C5 alkynyl, halo (C1-C5) alkyl, halo (C1-C5) alkoxy, C2-C5 alkenyloxy, C1-C5 alkoxy (C1-C5) alkoxy, piperidyl, nitro, or morpholinyl;
R₁₀ is C1-C5 alkyl, halo (C1-C5) alkyl, or C2-C5 alkenyl; and
R₁₁ and R₁₂ are independently hydrogen, halogen, or methyl.

15. The compound according to claim 14, an isomer, or a pharmaceutically acceptable salt thereof;
wherein,
R₁, R₂, R₅, R₆, R₈, and R₉ are hydrogen;
R₃ and R₄ are independently hydrogen, fluoro, chloro, cyano, methyl, ethenyl, ethynyl, or trifluoromethyl;
R₇ is t-butyl, trifloromethyl, methyl, chloro, bromo, nitro, or morpholinyl; and
R₁₀ is methyl or ethenyl.

16. The compound according to claims 14 or 15, an isomer, or a pharmaceutically acceptable salt thereof;
wherein,
R₁, R₂, R₅, R₆, R₈, and R₉ are hydrogen;
R₃ and R₄ are independently hydrogen, fluoro, chloro, cyano, methyl, ethenyl, ethynyl, or trifluoromethyl;
R₇ is t-butyl, trifluoromethyl, methyl, chloro, bromo, nitro, or morpholinyl; and
R₁₀ is methyl.

17. A pharmaceutical composition comprising the compound according to any one of claims 1 to 16, an isomer thereof, or a pharmaceutically acceptable salt thereof, as an active ingredient and a pharmaceutically acceptable carrier.

18. A pharmaceutical composition for preventing or treating a condition associated with the pathological stimulation and/or aberrant expression of vanilloid receptors, wherein said composition comprises the compound according to any one of claims 1 to 16, an isomer thereof, or a pharmaceutically acceptable salt thereof; and a pharmaceutically acceptable carrier.

19. The pharmaceutical composition according to claims 17 or 18, for treating a condition selected from the group consisting of pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis, and pancreatitis.

20. The pharmaceutical composition according to claim 19 wherein the pain is or is associated with a condition selected from the group consisting of osteoarthritis, rheumatoid arthritis, ankylosing spondylitis, diabetic neuropathic pain, post-operative pain, dental pain, non-inflammatory musculoskeletal pain (including fibromyalgia, myofascial pain syndrome and back pain), migraine and other types of headaches.

21. The pharmaceutical composition according to anyone of claims 17 to 20 **characterized in that** it is adapted for oral administration.

22. A method for inhibiting vanilloid ligand from binding to vanilloid receptor in a patient, comprising contacting cells expressing vanilloid receptor in the patient with the compound according to any one of claims 1 to 16, an isomer thereof, or a pharmaceutically acceptable salt thereof.

23. A method for preventing or treating a condition selected from the group consisting of pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis, and pancreatitis, which comprises administering to a mammal including a person in need thereof a therapeutically effective amount of the compound according to any one of claims 1 to 16, an isomer thereof, or a pharmaceutically acceptable salt thereof.

24. The method according to claim 23, wherein the pain is or is associated with a condition selected from the group consisting of osteoarthritis, rheumatoid arthritis, ankylosing spondylitis, diabetic neuropathic pain, post-operative pain, dental pain, non-inflammatory musculoskeletal pain (including fibromyalgia, myofascial pain syndrome and back pain), migraine, and other types of headaches.

25. Use of the compound according to any one of claims 1 to 16, an isomer thereof, or a pharmaceutically acceptable salt thereof, in preparation of a medicament for prevention or treatment of a condition that is associated with the aberrant expression and/or aberrant activation of a vanilloid receptor.

26. Use of the compound according to any one of claims 1 to 16, an isomer thereof, or a pharmaceutically acceptable salt thereof, in preparation of a medicament for prevention or treatment of a condition that is selected from the group consisting of pain, inflammatory disease of the joints, neuropathies, HIV-related neuropathy, nerve injury, neurodegeneration, stroke, urinary bladder hypersensitivity including urinary incontinence, cystitis, stomach duodenal ulcer, irritable bowel syndrome (IBS) and inflammatory bowel disease (IBD), fecal urgency, gastro-esophageal reflux disease (GERD), Crohn's disease, asthma, chronic obstructive pulmonary disease, cough, neurotic/allergic/inflammatory skin disease, psoriasis, pruritus, prurigo, irritation of skin, eye or mucous membrane, hyperacusis, tinnitus, vestibular hypersensitivity, episodic vertigo, cardiac diseases such as myocardial ischemia, hair growth-related disorders such as effluvium, alopecia, rhinitis and pancreatitis.

27. Use of the compound according to claim 26, wherein the condition is pain, which is or which is associated with a condition selected from the group consisting of osteoarthritis, rheumatoid arthritis, ankylosing spondylitis, diabetic neuropathic pain, post-operative pain, dental pain, non-inflammatory musculoskeletal pain (including fibromyalgia, myofascial pain syndrome and back pain), migraine, and other types of headaches.

28. A process for preparing a compound represented by the formula (III) 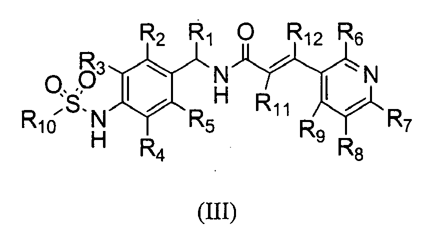 which comprises reacting a compound represented by the formula (IIIa); 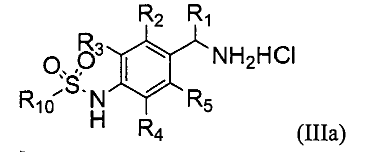 with a compound represented by the formula (IIIb); 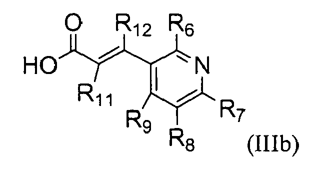 wherein,
R₁ is hydrogen or C1-C5 alkyl;
R₂, R₃, R₄, and R₅ are independently hydrogen, halogen, nitro, cyano, C1-C5 alkyl, C1-C5 alkoxy, halo (C1-C5) alkyl, C2-C5 alkenyl, C2-C5 alkynyl, carboxy, C1-C5 alkoxycarbonyl, or C1-C5 alkylthio;
R₆, R₇, R₈, and R₉ are independently hydrogen, hydroxy, halogen, nitro, carboxy, C1-C5 alkyl, C1-C5 alkoxy, C2-C5 alkenyl, C2-C5 alkynyl, halo (C1-C5) alkyl, halo (C1-C5) alkoxy, hydroxy (C1-C5) alkyl, C1-C5 alkylthio, C1-C5 alkylsulfonyl, C1-C5 alkylcarbonyl, C1-C5 alkoxycarbonyl, C2-C5 alkenyloxy, C1-C5 alkoxy (C1-C5) alkoxy, C1-C5 alkoxy (C1-C5) alkoxy (C1-C5) alkyl, C1-C3 alkylpiperazinyl, piperidyl, C1-C5 alkoxy (C1-C5) alkylamino, C1-C7 alkylamino, di(C1-C5 alkyl)amino, C3-C6 cycloalkyl which may be unsubstituted or substituted with one or more C1-C3 alkyl groups, pyrrolidinyl, phenyl, or morpholinyl, wherein the phenyl may be unsubstituted or substituted with one or more substituents selected from halogen, C1-C5 alkyl, and halo (C1-C5) alkyl;
R₁₀ is C1-C5 alkyl, halo (C1-C5) alkyl, or C2-C5 alkenyl; and
R₁₁ and R₁₂ are independently hydrogen, C1-C5 alkyl, or halogen.

29. The process according to claim 28, wherein the reaction is conducted in the presence of a coupling agent.

30. The process according to claim 29, wherein the coupling agent is selected from the group consisting of DCC (N,N-dicyclcohexylcarbodidimide), EDCI { 1-(3-dimethylaminopropyl)-3-ethylcarbodidimide hydrochloride (EDCI)}, and DMTMM {4-(4,6-dimethoxy-1,3,5-triazin-2-yl)-4-methylmorpholinium chloride}.

31. A process for preparing a compound represented by the formula (IIIc); 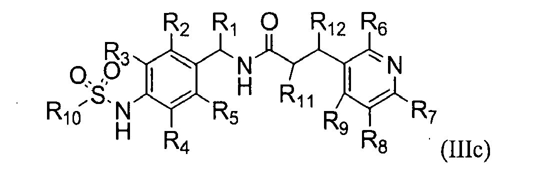 which comprises an reducing step of the compound of the formula (III).

32. The process according to claim 31, wherein the reducing step is conducted in the presence of hydrogen gas and palladium on carbon.

33. The process according to claims 28 to 32, wherein R₁, R₁₁, and R₁₂ are hydrogen.

34. The process according to claims 28 to 31, wherein, R₁, R₂, R₈, R₁₁, and R₁₂ are hydrogen; R₃ is hydrogen, fluoro, chloro, bromo, cyano, methyl, ethyl, ethenyl, ethynyl, or trifluoromethyl; R₄ and R₅ are independently hydrogen, fluoro, chloro, cyano, methyl, ethyl, or trifluoromethyl; R₆ is hydrogen, fluoro, chloro, bromo, methyl, methoxy, diethylamino, pyrrolidinyl, piperidyl, or morpholinyl; R₇ is isopropyl, t-butyl, or trifluoromethyl; R₉ is hydrogen or trifluoromethyl; and R₁₀ is methyl.
